(19) Europäisches Patentamt
European Patent Office
Office européen des brevets

(11) EP 1 227 149 A1

(12) EUROPEAN PATENT APPLICATION
published in accordance with Art. 158(3) EPC

(43) Date of publication:
31.07.2002 Bulletin 2002/31

(21) Application number: 00970079.0

(22) Date of filing: 26.10.2000

(51) Int Cl.[7]: C12N 15/12, C12N 5/10, C12P 21/02, C07K 16/18, C07K 14/47, G01N 33/50, G01N 33/15, A61K 38/17

(86) International application number:
PCT/JP00/07480

(87) International publication number:
WO 01/31004 (03.05.2001 Gazette 2001/18)

(84) Designated Contracting States:
AT BE CH CY DE DK ES FI FR GB GR IE IT LI LU MC NL PT SE
Designated Extension States:
AL LT LV MK RO SI

(30) Priority: 27.10.1999 JP 30518799
04.11.1999 JP 31345399

(71) Applicant: Takeda Chemical Industries, Ltd.
Osaka-shi, Osaka 541-0045 (JP)

(72) Inventor: SUGINO, Hiromu
Tokushima-shi, Tokushima 770-8041 (JP)

(74) Representative: Lewin, John Harvey
Takeda Patent Office,
11-12 Charles II Street
London SW1Y 4QU (GB)

# (54) NOVEL PROTEIN AND USE THEREOF

(57) The protein of the present invention, a partial peptide thereof or a salt thereof, expressed specifically in the brain, having PDZ domains and having affinity particularly for an activin receptor and/or an activin intracellular information transmission molecule, is useful for determining a binding protein having an ability to bind to said protein, for screening a compound inhibiting or promoting a binding of the protein of the present invention, etc. to the binding protein, and the like.

EP 1 227 149 A1

## Description

## Technical Field

The present invention relates to a novel protein having a specific amino acid sequence, a DNA comprising a DNA region encoding said protein, a method for producing the protein, and use of the protein and the DNA.

## Background Art

Heretofore, a large number of physiologically active substances have been isolated and identified, and their functions are being elucidated. Some of them are known to exhibit various activities in a wide variety of organs or cells. The various physiological activities in a wide variety of organs or cells appear generally via receptors to which the physiologically active substances bind, but there are many cases where whether the combination of the physiologically active substances binding to the receptors is identical in all organs and cells or specific to each organ or cell is not elucidated.

Some physiologically active proteins have PDZ domains. These domains are protein-binding domain modules found relatively recently. Accordingly, the in vivo distribution, functions and regulatory mechanism of proteins having PDZ domains are still not elucidated in many cases. The proteins having PDZ domains are considered to play an important role in formation of a backing structure of cell membrane, in formation of a network of cytoskeleton, and in formation of an intracellular signal transmission network developed in an intracellular cortex. In the nervous system, these proteins participate in forming complexes of neurotransmitter receptors, ion channels, etc., thus making them essential for assembly of protein clusters in synapse sites. Further, PDZ proteins whose expression is regulated depending on synaptic plasticity have been recently found, and there is the possibility that the PDZ proteins participate in alteration of the morphology of synapses accompanying plasticity through rearrangement of receptors, and they are considered to participate in construction of a nervous network at a development stage and in in vivo high-order mechanism in the brain. The PDZ domain is similar in certain respects to known peptide-binding domains, but has distinctive features. The PDZ domain is a domain preserved widely in microorganisms, higher plants and animals, and in many cases, the PDZ domain recognizes a C-terminal short amino acid sequence of its target proteins, and these target proteins are often membrane-penetrating receptors or channels. The PDZ domain is often found in a form repeated about 2- to 6-times in the same protein, and unlike other domain modules, some of the PDZ domains form homodimers. These are features important for participating in formation of a protein-crosslinked network and microdomains in intercellular adhesion, such as a cellular surface structure, tight junction of synapses, etc.

Activin is a regulatory factor inducing the mesoblast at the early stage of embryonic development, regulating the nervous induction and promoting secretion of follicle-stimulating hormone (FSH) from the anterior pituitary. It is a member of TGF-β super family. There are two types of receptors having the activity of serine/threoninekinase, type-I and type-II as a TGF-β family receptor. A ligand stimulus causes an activation of type-I by type-II. From the activated type-I, activin intracellular information transmission molecules (e.g., Smad) mediate an information transmission. There are a variety of molecules produced by splicing selectively from two kinds of genes, IIA and IIB for activin receptor type-II. It is expected to play an important role besides the activation of type-I, but, it has not been elucidated yet. Heretofore, it has been considered that the regulation of secretion of gonadotrophic hormone from the pituitary is mainly based on steroid hormone produced in the gonad, and since activin and its antagonist i.e. inhibin were found, this regulation attracts attention as a new hormone secretion-regulating mechanism in a hypothalamo-hypophysial-germ system. As analysis of the physiological activities of activin proceeds, this system was found to have various physiological activities including not only secretory regulation of FSH but also an activity of inducing or inhibiting differentiation of cells in a blood-corpuscle system or in reproductive cells, an activity of maintaining the viability of nerve cells, etc., but many features of this mechanism have been not elucidated in detail.

As a means of elucidating the physiological activities of a protein having PDZ domains and receptors (e.g., activin receptors) having an ability to bind to said protein, as well as of elucidating detailed molecular mechanisms for the expression of the physiological activities such as inhibition of differentiation of cells and neurotrophic factor-like activities in an activin-activin receptor system, etc., the object of the present invention is to provide a method for isolating said novel protein, a method for detecting the same, a DNA comprising a gene for said novel protein, a method for producing a protein encoded by the gene for said novel protein, and use of the DNA and the protein.

## Disclosure of Invention

To solve the problem described above, the present inventors made extensive study, and as a result, they screened a binding protein from a mouse brain cDNA library by using a yeast two-hybrid method in which an intracellular region in a mouse activin IIA receptor protein shown in SEQ ID NO:1 was used as a bait, thus isolating a cDNA clone which could be confirmed to bind to activin IIA receptors even in COS7 cells. Further, they analyzed said cDNA clone and found that said cDNA clone is a gene containing a region encoding 1 PDZ domain. Said protein encoded by this gene is a protein factor having domains for protein-protein interaction, and this protein binds, via said domains, to intracellular regions in activin receptors, but does not inhibit a signal transmission of activin.

Further, the present inventors extracted poly (A) $^+$RNA from a wide variety of mouse organs, and used a DNA shown in SEQ ID NO:4 as a probe to examine expression of the novel protein of the present invention by Northern hybridization, and as a result, they found that expression thereof occurs widely in a variety of organs such as the heart, the brain, the liver, the skeletal muscle, the kidney, the testis and the like. From this, said novel protein is suggested to play an important role (e.g., a transportation of activin receptor to a cell membrane) in the activin-activin receptor information transmission system and found to be applicable to diagnosis, treatment, etc. of diseases related to an abnormality of the function of activin receptor.

As further examination on the basis of these findings, the present inventors arrived at the present invention.

That is, the present invention provides:

(1) a protein which has an amino acid sequence identical to or substantially identical to an amino acid sequence represented by SEQ ID NO:2, or a salt thereof,

(2) a partial peptide of the protein according to item (1), or a salt thereof,

(3) a recombinant DNA which comprises a DNA encoding the protein according to item (1),

(4) the DNA according to item (3), which has a nucleotide sequence represented by SEQ ID NO:3, or a nucleotide sequence hybridizing therewith under high stringent conditions.

(5) a recombinant DNA which comprises a DNA encoding the partial peptide according to item (2).

(6) a recombinant vector comprising the DNA according to item (3),

(7) a transformant comprising the recombinant vector according to item (6),

(8) a method for producing the protein according to item (1) or a salt thereof, which comprises culturing the transformant according to item (7) to produce and accumulate the protein according to item (1), and then recovering the product,

(9) an antibody against the protein according to item (1), the partial peptide according to item (2) or a salt thereof,

(10) a method for quantifying the protein according to item (1), the partial peptide according to item (2) or a salt thereof, which comprises allowing a test solution containing the protein according to item (1), the partial peptide according to item (2) or a salt thereof and the labeled protein according to item (1), the labeled partial peptide according to item (2) or a labeled salt thereof to react competitively with the antibody according to item (9),

(11) a method for determining a binding protein to the protein according to item (1), the partial peptide according to item (2) or a salt thereof, which comprises using the protein according to item (1), the partial peptide according to item (2) or a salt thereof,

(12) a protein or a salt thereof which is obtained by the method according to item (11), which binds to the protein according to item (1), the partial peptide according to item (2) or a salt thereof,

(13) a method for screening a compound or a salt thereof inhibiting or promoting a binding of the protein according to item (1), the partial peptide according to item (2) or a salt thereof to the protein according to item (12) or a salt thereof, or an activin receptor protein or a salt thereof,

(14) the method for determining a protein according to item (11) or the screening method according to item (13), which comprises using the two-hybrid method,

(15) a kit for screening a compound or a salt thereof inhibiting or promoting a binding of the protein according to item (1), the partial peptide according to item (2) or a salt thereof to the protein according to item (12) or a salt thereof, or an activin receptor protein or a salt thereof, which comprises the protein according to item (1), and the partial peptide according to item (2) or a salt thereof,

(16) a compound or a salt thereof which inhibits or promotes a binding of the protein according to item (1), the partial peptide according to item (2) or a salt thereof to the protein according to item (12) or a salt thereof, or an activin receptor protein or a salt thereof, which is obtained by using the screening method according to item (13) or by using the screening kit according to item (15),

(17) a medicament comprising the protein according to item (12), the compound according to item (16) or a salt thereof,

(18) the medicament according to item (17), which is an agent for preventing or treating an abnormality in a nerve cell or a cerebral disease correlated with the protein according to item (12) or a salt thereof, an activin receptor or an activin intracellular information transmission molecule,

(19) a method for preventing or treating an abnormality in a nerve cell or a cerebral disease correlated with the protein according to item (12) or a salt thereof, an activin receptor or an activin intracellular information transmission molecule, which comprises administering an effective amount of the protein according to item (12), the compound according to item (16) or a salt thereof to a mammal, and

(20) use of the protein according to item (12), the compound according to item (16) or a salt thereof for preparing an agent for preventing or treating an abnormality in a nerve cell or a cerebral disease correlated with the protein according to item (12) or a salt thereof, activin receptors or activin intracellular information transmission molecules.

Further, the present invention provides:

(21) the protein according to item (1), which is a protein having PDZ domain,
(22) the protein according to item (21), which is a protein expressed in the heart, the brain, the liver, the skeletal muscle, the kidney, the testis, the lung or the spleen,
(23) the protein according to item (22), which is a protein having binding ability to activin receptors,
(24) the protein according to item (23), which is a protein which does not inhibit the signal transmission of activin,
(25) the protein according to item (1) or a salt thereof, wherein the protein comprises an amino acid sequence represented by SEQ ID NO:2, an amino acid sequence in which 1 or 2 or more (preferably 2 to 10) amino acid sequences in an amino acid sequence represented by SEQ ID NO:2 are deleted, an amino acid sequence in which 1 or 2 or more (preferably 2 to 10) amino acid sequences are added or inserted into an amino acid sequence represented by SEQ ID NO:2, or an amino acid sequence in which 1 or 2 or more (preferably 2 to 10) amino acid sequences in an amino acid sequence represented by SEQ ID NO:2 are substituted with other amino acids,
(26) the two-hybrid method according to item (14), wherein a yeast is used,
(27) a method for screening a compound or a salt thereof inhibiting or promoting a binding of the protein according to item (1) or a salt thereof to an activin receptor, which comprises comparing the case where a labeled protein according to item (1) or a salt thereof is contacted with an activin receptor, with the case where a labeled protein according to item (1) or a salt thereof and a test compound are contacted with the activin receptor, by measuring the amount of the labeled protein according to item (1) or a salt thereof bound to the activin receptor in both the cases,
(28) a method for screening a compound or a salt thereof inhibiting or promoting a binding of the protein according to item (1) or a salt thereof to the protein according to item (12) or a salt thereof, which comprises comparing the case where a labeled protein according to item (1) or a salt thereof is contacted with the protein according to item (12) or a salt thereof, with the case where the labeled protein according to item (1) or a salt thereof and a test compound are contacted with the protein according to item (12) or a salt thereof, by measuring the amount of the labeled protein according to item (1) or a salt thereof bound to the protein according to item (12) or a salt thereof in both the cases,
(29) a method for screening a compound or a salt thereof inhibiting or promoting a binding of the protein according to item (1) or a salt thereof to an activin receptor, which comprises comparing the case where the protein according to item (1) or a salt thereof is introduced into a cell expressing an activin receptor, with the case where the protein according to item (1) or a salt thereof and a test compound are introduced into the cell expressing the activin receptor, by measuring the amount of a binding of the protein according to item (1) or a salt thereof to the activin receptor in the cell in both the cases,
(30) a method for screening a compound or a salt thereof inhibiting or promoting a binding of the labeled protein according to item (1) or a salt thereof to an activin receptor, which comprises comparing the case where a labeled protein according to item (1) or a salt thereof is contacted with a membrane fraction of a cell expressing the activin receptor, with the case where the labeled protein according to item (1) or a salt thereof and a test compound are contacted with the membrane fraction of the cell expressing the activin receptor, by measuring the amount of the labeled protein according to item (1) or a salt thereof bound to the membrane fraction of the cell in both the cases,
(31) a method for screening a compound or a salt thereof inhibiting or promoting a binding of the protein according to item (1) or a salt thereof to an activin receptor, which comprises comparing the case where the protein according to item (1) or a salt thereof is introduced into a cell expressing an activin receptor, with the case where the protein according to item (1) or a salt thereof and a test compound are introduced into the cell expressing the activin receptor, by measuring the cell-stimulating activity via the activin receptor in both the cases,
(32) a compound obtained by the screening method according to any of items (27) to (31), or a salt thereof,
(33) a pharmaceutical composition comprising the compound according to item (32) or a salt thereof, and
(34) the medicament according to item (33), which is an agent for preventing or treating abnormalities in nerve cells or cerebral diseases correlated with the protein according to item (12) or a salt thereof, activin receptors or activin intracellular information transmission molecules.

Further, the present invention provides:

(35) non-human mammals having the extraneous DNA according to item (3) or a variant DNA thereof,
(36) the non-human mammals according to item (35) wherein the non-human mammals are rodents,

(37) the non-human mammals according to item (36) wherein the rodents are mice,
(38) the non-human mammals according to item (36) wherein the rodents are rats, and
(39) a recombinant vector comprising the DNA according to item (3) or a variant DNA thereof and capable of expression in mammals.

**Brief Description of Drawings**

Fig. 1 shows the entire nucleotide sequence of cDNA encoding the novel protein of the present invention, as well as an amino acid sequence (SEQ ID NO:2) encoded thereby.
Fig. 2 shows the entire nucleotide sequence of cDNA encoding the novel protein of the present invention, as well as an amino acid sequence (SEQ ID NO:2) encoded thereby. Fig. 2 is a continuation of Fig. 1.

**Best Mode for Carrying Out the Invention**

The protein comprising an amino acid sequence substantially identical to the amino acid sequence represented by SEQ ID NO:2 of the present invention may be a protein derived from cells (e.g., hepatic cells, spleen cells, nerve cells, glia cells, pancreatic β-cells, bone marrow cells, mesangial cells, Langerhans' cells, epidermal cells, epithelial cells, endothelial cells, fibroblasts, fibrous cells, muscular cells, fat cells, immune cells (e.g., macrophage, T cell, B cell, natural killer cell, mast cell, neutrophils, basophils, eosinophils, monocyte, etc.), megakaryocyte, synovial membrane cells, soft-bone cells, bone cells, osteoblast, osteoclast, mammary gland cells or interstitial cells, or their precursor cells, stem cells or cancer cells, etc.) or any tissues having such cells, for example, the brain and each site of brain (e.g., olfactory bulb, tonsil nuclei, cerebral basal bulb, hippocampus, thalamus, hypothalamus, cerebral cortex, medulla bulb, cerebellum, etc.), spinal cord, pituitary, stomach, pancreas, kidney, liver, gonads, thyroid glands, galls, bone marrow, adrenals, skin, muscles, lungs, digestive tracts (e.g., large and small intestines etc.), blood vessels, heart, thymus, spleen, salivary glands, peripheral blood, prostate, testicles, ovary, placenta, uterus, bone, joints, skeletal muscles, etc.], or cells in the hemocyte system or their cultured cell strain (particularly in the brain), from humans or warm-blooded animals (e.g., guinea pig, rat, mouse, chicken, rabbit, pig, sheep, cow, monkey, etc.), as well as a synthetic protein.
As the protein comprising an amino acid sequence substantially identical to an amino acid sequence represented by SEQ ID NO:2, an amino acid sequence having about 70% or more, preferably about 80% or more, more preferably about 90% or more, and most preferably about 95% or more homology with an amino acid sequence represented by SEQ ID NO:2, is exemplified.
The protein of the present invention comprising an amino acid sequence substantially identical to an amino acid sequence represented by SEQ ID NO:2 is preferably, for example, a protein having an amino acid sequence substantially identical to the amino acid sequence represented by SEQ ID NO:2 and having substantially identical physiological properties with those of a protein having the amino acid sequence represented by SEQ ID NO:2.
As the substantially identical physiological properties, for example, qualitative elements such as receptor affinity, signal transmission ability, specificity of distribution of expression in organs, etc. are exemplified. The term "substantially identical" means that these physiological activities are biologically or physiologically homogenous. Therefore, although it is preferable that the activities such as the strength of receptor affinity be equal (e.g., about 0.1- to 20-fold, preferably about 0.5- to 2-fold), quantitative elements such as level of these activities and the molecular weight of the protein etc. may be different.
Measurement of the receptor affinity can be carried out according to the known method per se, for example, by the screening method described later.
In addition, as the protein of the present invention, for example, a mutein such as a protein comprising an amino acid sequence in which 1 or 2 or more (preferably about 1 to 10, more preferably about 1 to 5, further preferably about 1 to 3) amino acids in the amino acid sequence represented by SEQ ID NO:2 are deleted, an amino acid sequence in which 1 or 2 or more (preferably about 1 to 10, more preferably about 1 to 5, further preferably about 1 to 3) amino acids are added or inserted to the amino acid sequence represented by SEQ ID NO:2, or an amino acid sequence in which 1 or 2 or more (preferably about 1 to 10, more preferably about 1 to 5, further preferably about 1 to 3) amino acids in the amino acid sequence represented by SEQ ID NO:2 are substituted with other amino acids, or a protein containing a combination of these amino acid sequences, etc. are exemplified.
In proteins in the present specification, a left end is an N-terminal (amino terminal) and a right end is a C-terminal (carboxyl terminal) according to the convention of the peptide display. Although the proteins of the present invention including a protein comprising an amino acid sequence represented by SEQ ID NO:2 usually have carboxyl group (-COOH) or carboxylate ($-COO^-$) as a C-terminal, they may have an amide ($-CONH_2$) or an ester (-COOR) as a C-terminal.
As R in ester, for example $C_{1-6}$ alkyl group such as methyl, ethyl, n-propyl, isopropyl and n-butyl, $C_{3-8}$ cycloalkyl

group such as cyclopentyl and cyclohexyl, $C_{6-12}$ aryl group such as phenyl and α-naphthyl, and $C_{6-12}$ aryl-$C_{1-2}$ alkyl group such as benzyl, phenetyl and α-naphthylmethyl, as well as a pivaloyloxymethyl ester group generally used in an oral ester are used.

When the protein of the present invention has carboxyl group (or carboxylate) at a position other than a C-terminal, proteins in which carboxyl group is amidated or esterified are also included in the protein of the present invention. As an ester used in this case, for example, an ester at a C-terminal described above is used.

Further, the protein of the present invention includes proteins in which an amino group of a methionine residue of a N-terminal is protected with a protecting group (e.g., $C_{1-6}$ acyl group such as formyl group and acetyl group, etc.), proteins in which an N-terminal glutamic acid residue formed by the cleavage in the N-terminal side in the living body is converted into pyroglutamine, proteins in which, for example, -OH, COOH, $NH_2$, -SH, etc. on a side chain of an intramolecular amino acid is protected with a suitable protecting group (e.g., $C_{1-6}$ acyl group such as formyl group and acetyl group, etc.), and conjugated proteins such as glycoprotein having sugar chains bound thereto, etc.

As an embodiment of the protein of the present invention, for example, a protein comprising an amino acid sequence represented by SEQ ID NO:2, etc. is used.

As a partial peptide of the protein of the present invention, any peptides may be used as long as they are partial peptides of the protein of the present invention described above and having the physiological activities possessed by the protein of the present invention, for example, activities such as receptor affinity and an ability to transmit signal information, and specificity of distribution of expression thereof in organs, etc. For example, a peptide having 20 or more, preferably 50 or more, more preferably 80 or more, further preferably 100 or more and most preferably 120 or more amino acids from the amino acid sequence constituting the protein of the present invention and having receptor affinity and an ability to transmit signal information, etc., are used.

Further, in the partial peptide of the present invention, 1 or 2 or more (preferably about 1 to 10, more preferably about 1 to 3) amino acids in the amino acid sequence thereof may be deleted, 1 or 2 or more (preferably about 1 to 10, more preferably about 1 to 5, further preferably about 1 to 3) amino acids may be added to the amino acid sequence thereof, or 1 or 2 or more (preferably about 1 to 10, more preferably about 1 to 5, further preferably about 1 to 3) amino acids in the amino acid sequence thereof may be substituted with other amino acids.

Although the partial peptide of the present invention has usually carboxyl group (-COOH) or carboxylate ($-COO^-$) as a C-terminal, it may have an amide ($-CONH_2$) or an ester (-COOR) as a C-terminal, as with the protein of the present invention described above.

Further, the partial peptide of the present invention, similar to the protein of the present invention described above, includes peptides in which an amino group of a methionine residue as an N-terminal is protected with a protecting group, peptides in which an N-terminal glutamyl group formed by the cleavage in the N-terminal side in the living body is converted into pyroglutamine, peptides in which a substituent group on a side chain of an intramolecular amino acid is protected with a suitable protecting group, and conjugated peptides such as glycopeptides having sugar chains bound thereto.

As a salt of the protein of the present invention or of a partial peptide thereof, physiologically acceptable acid addition salts are particularly preferable. As such salts, for example, salts with inorganic acids (e.g., hydrochloric acid, phosphoric acid, hydrobromic acid, sulfuric acid) or salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid), etc., are used. Further, salts with inorganic bases (e.g., alkali metals such as sodium and potassium, etc., alkaline earth metals such as calcium and magnesium, etc., aluminum, ammonium, etc.), salts with organic bases (e.g., trimethylamine, triethylamine, pyridine, picoline, 2,6-lutidine, ethanolamine, diethanolamine, triethanolamine, cyclohexyl amine, dicyclohexyl amine, N,N'-dibenzylethylene diamine, etc.), etc., are also used.

The protein of the present invention or a salt thereof can be produced from cells or tissues of a human being or a warm blood mammal described above by the method known per se, or can be prepared by culturing a transformant containing a DNA encoding a protein described below. Alternatively, it can also be prepared according to a method of synthesizing a peptide described below.

For production from human or mammalian tissues or cells, the human or mammalian tissues or cells are homogenized and then extracted with, for example, an acid, and the extract can be subjected to purification and isolation by a combination of chromatographic techniques such as reverse-phase chromatography, ion-exchange chromatography, etc.

For production of the protein of the present invention, a partial peptide thereof or a salt thereof, or an amide derivative thereof and a commercial resin for protein synthesis can be used. Examples of such resin include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylphenylacetamidemethyl resin, polyacrylamide resin, 4-(2',4'-dimethoxyphenylhydroxymethyl)phenoxy resin, 4-(2',4'-dimethoxyphenyl-Fmocaminoethyl)phenoxy resin, etc. Using such resin, an amino acid wherein an a-amino group and a functional group of the side chain are suitably protected are condensed on a resin according to the order of sequence of the desired protein by various condensation methods known per se. At the end of the reaction, a protein is cut out from the resin, simultaneously with which each protecting group is removed, intramolecular disulfide bond is formed in a highly diluted solution, whereby the desired protein, a partial peptide thereof or an amide derivative thereof is obtained.

A wide variety of activating reagents usable for protein synthesis can be used for condensation of the protected amino acids described above, and carbodiimides are particularly preferable. Examples of such carbodiimides include DCC, N,N'-diisopropylcarbodiimide, N-ethyl-N'-(3-dimethylaminopropyl)-carbodiimide, etc. For activation by these reagents, the protected amino acids along with racemization inhibitors (e.g., HOBt, HOOBt) can be added to the resin directly or after the protected amino acids were activated in advance as symmetric acid anhydrides, HOBt esters or HOOBt esters.

The solvent used for activation of each protected amino acid or for condensation thereof with the resin can be selected as necessary from those solvents known to be usable in protein condensation reaction. Examples of such solvent include acid amides such as N,N-dimethylformamide, N,N-dimethylacetamide and N-methylpyrrolidone, halogenated hydrocarbons such as methylene chloride and chloroform, alcohols such as trifluoroethanol, sulfoxides such as dimethyl sulfoxide, ethers such as pyridine, dioxane and tetrahydrofuran, nitriles such as acetonitrile and propionitrile, esters such as methyl acetate and ethyl acetate, or a suitable mixture thereof. The reaction temperature is usually selected as necessary within the range known to be usable in the reaction for forming protein bonds, and usually the reaction temperature is selected within the range of about -20°C to 50°C. The activated amino acid derivatives are used usually in excess (1.5- to 4-fold). When their condensation is insufficient as a result of a test using ninhydrin reaction, their sufficient condensation is achieved by repeatedly carrying out the condensation reaction without conducting elimination of the protecting groups. When their sufficient condensation is not achieved even by repeatedly carrying out the reaction, the unreacted amino acids can be acetylated with acetic anhydride or acetyl imidazole.

The protecting groups for amino groups in the starting materials include, for example, Z, Boc, tert-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

The carboxyl group can be protected by, for example, alkyl esterification (e.g., straight-chain, branched or cyclic alkyl esterification such as methyl, ethyl, propyl, butyl, tert-butyl, cyclopentyl, cyclohexyl, cycloheptyl, cyclooctyl or 2-adamantyl esterification), aralkyl esterification (e.g., benzyl esterification, 4-nitrobenzyl esterification, 4-methoxybenzyl esterification, 4-chlorobenzyl esterification, benzhydryl esterification), phenacyl esterification, benzyloxycarbonylhydrazidation, tert-butoxycarbonylhydrazidation, tritylhydrazidation, etc.

The hydroxyl group in serine can be protected by, for example, esterification or etherification. A suitable group used in this esterification includes, for example, lower alkanoyl groups such as acetyl group, alloyl groups such as benzoyl group, and carbonic acid-derived groups such as benzyloxycarbonyl group and ethoxycarbonyl group. A suitable group for etherification includes, for example, benzyl group, tetrahydropyranyl group, tert-butyl group, etc.

The protecting group used for the phenolic hydroxyl group in tyrosine includes, for example, Bzl, $Cl_2$-Bzl, 2-nitrobenzyl, Br-Z, tert-butyl, etc.

The protecting group used for imidazole in histidine includes, for example, Tos, 4-methoxy-2,3,6-trimethylbenzenesulfonyl, DNP, benzyloxymethyl, Bum, Boc, Trt, Fmoc, etc.

Activated carboxyl groups in the starting materials include, for example, the corresponding acid anhydrides, azides and active esters (i.e. esters with alcohols such as pentachlorophenol, 2,4,5-trichlorophenol, 2,4-dinitrophenol, cyanomethyl alcohol, p-nitrophenol, HONB, N-hydroxysuccinamide and HOBt). The activated amino groups in the starting materials include, for example, the corresponding phosphoric amides.

Examples of methods for removing (leaving) the protecting groups include catalytic reduction in a hydrogen stream in the presence of a catalyst such as Pd-black or Pd-carbon, acid treatment with anhydrous hydrogen fluoride, methane sulfonic acid, trifluoromethane sulfonic acid, trifluoroacetic acid or a mixed solution thereof, base treatment using diisopropylethylamine, triethylamine, piperidine or piperazine, and reduction using sodium in liquid ammonia. The leaving reaction by the acid treatment described above is carried out generally at a temperature of about -20°C to 40°C, and it is effective in the acid treatment to add a cation scavenger such as anisole, phenol, thioanisole, m-cresol, p-cresol, dimethylsulfide, 1,4-butanedithiol and 1,2-ethanedithiol. A 2,4-dinitrophenyl group used as a protecting group for imidazole in histidine can also be removed by treatment with thiophenol, while a formyl group used as a protecting group for indole in tryptophan can be removed not only by deprotection by acid treatment in the presence of 1,2-ethanedithiol or 1,4-butanedithiol, but also by alkali treatment using dilute sodium hydroxide solution or dilute ammonia.

Protection and protecting groups for functional groups which should not participate in the reaction of the starting materials, elimination of the protecting groups, and activation of functional groups participating in the reaction can be selected as necessary from known groups or known means.

Another method of obtaining an amide derivative of the protein or a partial peptide thereof includes, for example, protecting the α-carboxyl group of a C-terminal amino acid by amidation, then extending a peptide (protein) chain at the side of the amino group until it attains desired chain length, and thereafter producing a protein (partial peptide) wherein only the protecting group for the N-terminal α-amino group of said peptide chain is removed and a protein (partial peptide) wherein only the protecting group for the C-terminal carboxyl group of said peptide chain is removed, followed by condensation both the proteins (partial peptides) in the mixed solvent as described above. The details of the condensation reaction are the same as those described above. The protected protein obtained by condensation is purified, and every protecting group is removed by the method descried above, whereby the desired crude protein (partial peptide) can be obtained. This crude protein (partial peptide) is purified by a wide variety of known purification techniques, and by lyophilizing its major fraction, the desired amide derivative of the protein (partial peptide) can be obtained.

To obtain an ester derivative of the protein or a partial peptide thereof, for example, the α-carboxyl group of a C-terminal amino acid is condensed with desired alcohol to form an amino acid ester from which the desired ester derivative of the protein (partial peptide) can be obtained in the same manner as for the amide derivative of the protein (partial peptide).

The partial peptide of the present invention or a salt thereof can be produced according to a peptide synthesis method known per se or by cleaving the protein of the present invention with a suitable peptidase. For example, the peptide synthesis method may be the solid- or liquid-phase synthesis method. That is, the desired peptide can be obtained by condensation of a partial peptide or amino acids capable of constituting the partial peptide of the present invention with the remainder, followed by elimination of protecting groups if any from the product. As the known condensation method and the elimination of the protecting groups, mention is made of, for example, the methods described in (1) to (5) below:

(1) M. Bodanszky and M. A. Ondetti, Peptide Synthesis, Interscience Publisher, New York (1966);
(2) Schroeder and Luebke, The Peptide, Academic Press, New York (1965) ;
(3) Nobuo Izumiya et al., Basis and Experiments in Peptide Synthesis (in Japanese), Maruzen Co., Ltd. (1975);
(4) Haruaki Yajima and Shunpei Sakakibara, Biochemical Experimental Course 1, Protein Chemistry IV, 205 (1977); and
(5) Haruaki Yajima (supervisor), Development of Medicines, a second series, vol. 14, Peptide Synthesis, Hirokawashoten.

After the reaction, the protein of the present invention or a partial peptide thereof can be isolated and purified by a combination of conventional purification techniques such as solvent extraction, distillation, column chromatography, liquid chromatography and recrystallization. If said protein or a partial peptide thereof is obtained in a free form by these methods, the product can be converted into a suitable salt by a known method, or if the product is obtained in a salt form, it can be converted into a free form by a known method.

As the DNA encoding the protein of the present invention, any DNAs may be used as long as they comprise a nucleotide sequence encoding the protein of the present invention described above. Further, the DNA may be any of a DNA isolated by the known method per se from a genomic DNA, a genomic DNA library, a cDNA derived form the aforementioned cells or tissues, a cDNA library derived from the aforementioned cells or tissues, and a synthetic DNA.

A vector used for a library may be any of bacteriophage, plasmid, cosmid and phagemide, etc. In addition, isolation can be performed directly by Reverse Transcriptase Polymerase Chain Reaction (abbreviated hereinafter to RT-PCR method) using total RNA or mRNA fraction prepared from the aforementioned cells or tissues.

As the DNA encoding the protein of the present invention, for example, (1) a DNA comprising a nucleotide sequence represented by SEQ ID NO:3 or (2) a DNA having a DNA hybridizing under high stringent conditions with a DNA having nucleotide sequence represented by SEQ ID NO:3 and encoding a protein having substantially homogenous physiological activities (for example activities such as receptor affinity and signal transmission ability, and specificity of distribution of expression thereof in organs, etc.) with those of a protein having an amino acid sequence represented by SEQ ID NO:2 may be used.

As the DNA which can hybridize, under high stringent conditions, with a nucleotide sequence represented by SEQ ID NO:3, for example, a DNA comprising a nucleotide sequence having about 70% or more, preferably about 80% or more, more preferably about 90% or more and most preferably about 95% or more homology with a nucleotide sequence represented by SEQ ID NO:3, etc. are used.

Hybridization can be carried out according to a known method per se or its analogous method, for example, a method described in Molecular Cloning, 2nd ed. (J. Sambrook et al., Cold Spring Harbor Lab. Press, 1989), etc. If a commercial library is used, hybridization can be carried out according to the manufacture's instructions. Preferably, hybridization can be conducted under high stringent conditions.

The high stringent conditions refer to those conditions under which the concentration of sodium is about 19 to 40 mM, preferably about 19 to 20 mM, and the temperature is about 50 to 70°C, preferably about 60 to 65°C. In particular, the case where the concentration of sodium is about 19 mM and the temperature is about 65°C is the most preferable.

More specifically, as a DNA encoding a protein comprising an amino acid sequence represented by SEQ ID NO:2, a DNA having a nucleotide sequence represented by SEQ ID NO:3, etc. are used.

As a DNA encoding the partial protein of the present invention, any DNAs may be used as long as they comprise a nucleotide sequence encoding the partial protein of the present invention described above. Further, said DNA may be any of a DNA isolated by the known method per se from a genomic DNA, a genomic DNA library, a cDNA derived form the aforementioned cells or tissues, a cDNA library derived from the aforementioned cells or tissues, and a synthetic DNA.

As a DNA encoding the partial peptide of the present invention, for example, (1) a DNA comprising a nucleotide sequence represented by SEQ ID NO:3 or (2) a DNA having a DNA hybridizing under high stringent conditions with a DNA having a nucleotide sequence represented by SEQ ID NO:3 and having a partial nucleotide sequence of a DNA encoding a protein having substantially homogenous physiological activities with those of a protein having an amino acid sequence represented by SEQ ID NO:2, etc. are used.

As the hybridization method and high stringent conditions, those described above are used.

As a means of cloning the DNA encoding the protein of the present invention or a partial peptide thereof (hereinafter referred to collectively as the protein of the present invention), it is possible to use amplification by the PCR method using synthetic DNA primers having a nucleotide sequence encoding a partial sequence of the protein of the present invention as well as isolation by hybridizing a DNA integrated in a suitable vector or a DNA isolated by the known method per se from a genomic DNA, a genomic DNA library, a cDNA derived from the aforementioned cells or tissues or a cDNA library derived from the aforementioned cells or tissues with a labeled DNA fragment or synthetic DNA encoding a part or the whole of the protein of the present invention. Hybridization can be carried out according to a method described in, for example, Molecular Cloning, 2nd ed. (J. Sambrook et al., Cold Spring Harbor Lab. Press, 1989). If a commercial library is used, hybridization can be carried out according to the manufacture's instructions.

Conversion of a nucleotide sequence of DNA can be carried out by a known method per se, for example, by ODA-LA PCR method, the Gupped duplex method or Kunkel method or its analogous method by using a known kit such as Mutant™-super Express Km (Takara Shuzo Co., Ltd.) or Mutant™-K (Takara Shuzo Co., Ltd.), etc.

A DNA encoding a cloned protein of the present invention can be used as it is depending on the object, or if desired, by digesting it with a restriction enzyme or adding a linker thereto. The DNA may have ATG as a translation initiation codon at the 5'-terminal thereof and TAA, TGA or TAG as a translation termination codon at the 3'-terminal thereof. These translation initiation and termination codons can also be added to the DNA via a suitable synthetic DNA adaptor.

An expression vector for the protein of the present invention can be produced for example by (A) cutting the desired DNA fragment off from the DNA encoding the protein of the present invention and then (B) ligating said DNA fragment to a region downstream from a promoter in a suitable expression vector.

The vector used includes E. coli-derived plasmid (e.g., pBR322, pBR325, pUC12, pUC13), Bacillus subtilis-derived plasmid (e.g., pUB110, pTP5, pC194), yeast-derived plasmid (e.g., pSH19, pSH15), bacteriophage such as λ-phage, and animal viruses such as retrovirus, vaccinia virus and Baculovirus, as well as pA1-11, pXT1, pRc/CMV, pRc/RSV, pcDNAI/Neo, etc.

The promoter used in the present invention may be any suitable promoter compatible with a host used for expression of the gene. For example, when animal cells are used as the host, mention is made of SV40-derived promoter, retrovirus promoter, metallothionein promoter, heat shock promoter, cytomegalovirus promoter, SRα promoter, etc. Among these, cytomegalovirus promoter, SRα promoter, etc. are preferably used. It is preferable to use trp promoter, lac promoter, recA promoter, $\lambda P_L$ promoter, lpp promoter, etc. for microorganisms of the genus Escherichia as the host, SPO1 promoter, SPO2 promoter, penP promoter, etc. for microorganisms of the genus Bacillus as the host, and pH05 promoter, PGK promoter, GAP promoter, ADH promoter, etc. for yeasts as the host. When insect cells are used as the host, polyhedron promoter, P10 promoter, etc. are preferable.

The expression vector may contain an enhancer, a splicing signal, a poly A-added signal, a selective marker, an SV40 origin of replication (also referred to hereinafter as SV40 ori), etc. in addition to the promoter described above. The selective marker includes, for example, dihydrofolate reductase (also referred to hereinafter as dhfr) gene [methotrexate (MTX) resistance], ampicillin resistance gene (also referred to hereinafter as $Amp^r$) and neomycin resistance gene (G418 resistance, also referred to hereinafter as Neo). In particular, if the dhfr gene is used as a selective marker for CHO ($dhfr^-$) cells, a transformant carrying the desired gene can also be selected in a thymidine-free medium.

A signal sequence compatible with the host is added as necessary to a nucleotide sequence for the N-terminal of the protein of the present invention. An alkali phosphatase signal sequence, Omp A signal sequence, etc. can be utilized for microorganisms of the genus Escherichia used as the host; an α-amylase signal sequence, subtilisin signal sequence, etc. for microorganisms of the genus Bacillus as the host; a mating factor α signal sequence, invertase signal sequence, etc. for yeasts as the host; and an insulin signal sequence, α-interferon signal sequence, antibody molecule signal sequence, etc. for animal cells as the host.

The thus constructed vector containing the DNA encoding the protein of the present invention can be used to produce transformants.

The microorganisms of the genus Escherichia, the microorganisms of the genus Bacillus, yeasts, insect cells, insects, animal cells, etc. are used as the host.

The microorganisms of the genus Escherichia used include, for example, Escherichia coli K12 DH1 (Proc. Natl. Acad. Sci. USA, 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)), etc.

The microorganisms of the genus Bacillus used include, for example, Bacillus subtilis MI114 (Gene, 24, 255 (1983)), 207-221 (Journal of Biochemistry, 95, 87 (1984)), etc.

The yeasts used include, for example, Saccharomyces cerevisiae AH22, AH22R$^-$, NA87-11A, DKD-5D, 20B-12, Schizosaccharomyces pombe NCYC1913, NCYC2036, Saccharomyces picjia pastoris, etc.

The insect cells used when the virus is AcNPV include, for example, cells (Spodoptera frugiperda cells; Sf cells) from an established cell line derived from caterpillars of Spodoptera frugiperda, MG1 cells derived from the midgut in Trichoplusia ni, High Five™ cells derived from eggs of Trichoplusia ni, cells derived from Mamestra brassicae and cells derived from Estigmena acrea. When the virus is BmNPV, cells (Bombyx mori N cells; BmN cells) from an established cell line derived from silkworms, etc., are used. The Sf cells used include, for example, Sf9 cells (ATCC CRL1711), Sf21 cells (Vaughn, J. L. et al., In Vivo, 13, 213-217 (1977)), etc.

The insects used include, for example, silkworm caterpillars (Maeda et al., Nature, 315, 592 (1985)).

The animal cells used include, for example, simian cell COS-7, Vero, Chinese hamster cell CHO, DHFR gene-defect Chinese hamster cell CHO (dhfr$^-$ CHO cells), mouse L cells, mouse AtT-20, mouse myeloma cells, rat GH3, human FL cells, etc.

The microorganisms of the genus Escherichia can be transformed according to a method described in e.g., Proc. Natl. Acad. Sci. USA, 69, 2110 (1972) or Gene, 17, 107 (1982), etc.

The microorganisms of the genus Bacillus can be transformed according to a method described in e.g., Molecular & General Genetics, 168, 111 (1979), etc.

The yeasts can be transformed according to a method described in e.g., Methods in Enzymology, 194, 182-187 (1991), Proc. Natl. Acad. Sci. USA, 75, 1929 (1978), etc.

The insect cells or insects can be transformed according to a method described in e.g., Bio/Technology, 6, 47-55 (1988), etc.

The animal cells can be transformed according to a method described in e.g., "Saibo Kogaku Bessatsu 8, Shin-Saibo Kogaku Jikken Protocol (Cell Technology, Extra Number 8, New Experimental Protocol in Cell Technology)", 263-267 (1995) (published by Shujunsha), Virology, 52, 456 (1973), etc.

Thereby, a transformant transformed with an expression vector comprising a DNA encoding the protein can be obtained.

When transformants derived from the microorganisms of the genus Escherichia or Bacillus as the host are cultured, the medium used for their culture is preferably a liquid medium containing a carbon source, a nitrogen source, mineral, etc. necessary for growth of the transformants. The carbon source includes, for example, glucose, dextrin, soluble starch, sucrose, etc.; the nitrogen source includes, for example, inorganic or organic materials such as ammonium salts, nitrates, corn steep liquor, peptone, casein, meat extract, soybean cake and potato extract, etc.; and the mineral includes, for example, calcium chloride, sodium dihydrogen phosphate, magnesium chloride, etc. In addition, yeast extracts, vitamins, growth promoters, etc. may be added. The pH value of the medium is desirably about 5 to 8.

For example, the medium for culturing the microorganisms of the genus Escherichia is preferably M9 medium containing glucose and casamino acid (Miller, Journal of Experiments in Molecular Genetics, 431-433, Cold Spring Harbor Laboratory, New York, 1972). To permit the promoter to work efficiently, a chemical such as 3β-indolylacrylic acid can be added as necessary.

The transformants from the microorganisms of the genus Escherichia as the host are cultured usually at about 15 to 43°C for about 3 to 24 hours during which the medium may be aerated or stirred as necessary.

The transformants from the microorganisms of the genus Bacillus as the host are cultured usually at about 30 to 40°C for about 6 to 24 hours during which the medium may be aerated or stirred as necessary.

The medium used for culturing the transformants from yeasts as the host includes, for example, Burkholder minimum medium (Bostian, K. L. et al., Proc. Natl. Acad. Sci. USA, 77, 4505 (1980)) and SD medium containing 0.5% casamino acid (Bitter, G. A. et al., Proc. Natl. Acad. Sci. USA, 81, 5330 (1984)). The pH value of the medium is adjusted preferably to about 5 to 8. The transformants are cultured usually at about 20 to 35°C for about 24 to 72 hours during which the medium may be aerated or stirred as necessary.

The medium used for culturing the transformants from insect cells or insects as the host includes, for example, a medium prepared by adding additives such as 10% inactivated bovine serum as necessary to Grace's insect medium (Grace, T. C. C., Nature, 195, 788 (1962)). The pH value of the medium is adjusted preferably to about 6.2 to 6.4. The transformants are cultured usually at about 27°C for about 3 to 5 days during which the medium may be aerated or stirred as necessary.

The medium used for culturing the transformants from animal cells as the host includes, for example, MEM medium containing about 5 to 20% fetal bovine serum (Science, 122, 501 (1952)), DMEM medium (Virology, 8, 396 (1959)), RPMI 1640 medium (Journal of the American Medical Association, 199, 519 (1967)), 199 medium (Proceeding of the Society for the Biological Medicine, 73, 1 (1950)), etc. The pH value is preferably about 6 to 8. The transformants are cultured usually at about 30 to 40°C for about 15 to 60 hours during which the medium may be aerated or stirred as necessary.

As described above, the protein of the present invention can be formed in the culture medium or in the transformants.

Separation and purification of the protein of the present invention from the above culture can be performed for example by the following method.

To extract the protein of the present invention from the cultured microorganisms or cells, the cultured microorganisms or cells are collected in a usual manner, suspended in a suitable buffer, disrupted by sonication, lysozyme and/or freezing and thawing, and centrifuged or filtered to give a crude extract of the protein. The buffer may contain protein denaturants such as urea and guanidine hydrochloride and surfactants such as Triton X-100™. When the protein is secreted into the culture liquid, the culture supernatant is collected by separating the supernatant from the cultured microorganisms or cells by a known method per se.

The culture supernatant thus obtained, or the protein contained in the extract, can be purified by a suitable combination of separation and purification techniques known per se. As these known separation and purification techniques, a method of utilizing solubility such as salting-out and solvent precipitation, a method of mainly utilizing a difference in molecular weight such as dialysis, ultrafiltration, gel filtration and SDS-polyacrylamide gel electrophoresis, a method of utilizing a difference in electric charge such as ion-exchange chromatography, a method of utilizing specific affinity such as affinity chromatography, a method of utilizing a difference in hydrophobicity such as reverse-phase high performance liquid chromatography, a method of utilizing a difference in isoelectric point such as isoelectric focusing can be used.

If the protein thus obtained is in a free form, it can be converted into a salt by a known method per se or its analogous method, while if the resulting protein is obtained in the form of a salt, it can be converted into a free protein or another salt by a known method per se or its analogous method.

Before or after purification, the protein produced by the transformants can be optionally modified or its partial polypeptide can be removed by allowing a suitable protein-modifying enzyme to act on the protein. For example, trypsin, chymotrypsin, arginyl endopeptidase, protein kinase or glycosidase, etc. is used as the protein-modifying enzyme.

The thus formed protein of the present invention or a salt thereof can be measured for its activity by an experiment of binding to a labeled ligand, enzyme immunoassays using specific antibody, etc.

The antibody against the protein of the present invention, a partial peptide thereof or a salt thereof may be a polyclonal or monoclonal antibody capable of recognizing the protein of the present invention, a partial peptide thereof or a salt thereof.

The antibody against the protein of the present invention, a partial peptide thereof or a salt thereof (referred to collectively as the protein of the present invention) can be produced by a known method for producing antibody or antiserum by using the protein of the present invention as the antigen.

[Preparation of a monoclonal antibody]

(a) Preparation of monoclonal antibody-producing cells

The protein of the present invention is administered alone or together with a carrier and a diluent into warm-blooded animals at a site where the antibody can be produced by administration. To enhance the ability of the animals upon administration to produce the antibody, complete Freund's adjuvant or incomplete Freund's adjuvant may be administered. Administration is conducted usually once every 2 to 6 weeks and about 2 to 10 times in total. The warm-blooded animals used include, for example, monkey, rabbit, dog, guinea pig, mouse, rat, sheep, goat and chicken, among which mouse and rat are preferably used.

For production of the monoclonal antibody-producing cells, those animals having antibody titer are selected from the warm-blooded animals (e.g., mice) immunized with the antigen, and on the second to fifth day after the final immunization, their spleens or lymph nodes are collected, and the antibody-producing cells contained therein are fused with myeloma cells, whereby monoclonal antibody-producing hybridoma can be produced. The antibody titer in antiserum can be measured for example by reacting the antiserum with the labeled protein described below and then measuring the activity of the labeling agent bound to the antibody. Fusion can be carried out by a known method such as the method of Keller and Millstein (Nature, 256, 495 (1975)). The fusion promoter includes polyethylene glycol (PEG) and Sendai virus, and PEG is preferably used.

The myeloma cells include, for example, NS-1, P3U1, SP2/0 and AP-1, among which P3U1 is preferably used. The ratio of the antibody-producing cells (spleen cells) to the myeloma cells used is preferably from about 1 : 1 to about 20 : 1, and cell fusion can be effected efficiently by incubating the cells for 1 to 10 minutes at 20 to 40°C, preferably 30 to 37°C, in the presence of PEG (preferably PEG 1000 to PEG 6000) at a concentration of about 10 to 80%.

The anti-protein antibody-producing hybridoma can be screened by various methods, for example, by adding a culture supernatant of the hybridoma to a solid phase (e.g., a microplate) having the antigenic protein adsorbed thereon directly or along with a carrier and then adding a radioactive substance- or enzyme-labeled anti-immunoglobulin antibody (which is an anti-mouse immunoglobulin antibody when mouse cells are subjected to cell fusion) or protein A to detect the anti-protein monoclonal antibody bound to the solid phase or by adding a culture supernatant of the hybridoma to a solid phase having an anti-immunoglobulin antibody or protein A adsorbed thereon and then adding the protein labeled with a radioactive substance or with an enzyme to detect the anti-protein monoclonal antibody bound to the solid phase.

The anti-protein monoclonal antibody can be screened according to a known method per se or its analogous method. Screening can be carried out usually in an animal cell culture medium to which HAT (hypoxanthine, aminopterin, thymidine) was added. The screening and breeding medium may be any medium in which the hybridoma can grow. Examples of such medium include RPMI 1640 medium containing 1 to 20% (preferably 10 to 20%) fetal bovine serum, GIT medium containing 1 to 10% fetal bovine serum (Wako Pure Chemical Industries, Ltd.), and a serum-free medium for hybridoma culture (SFM-101, Nissui Pharmaceutical, Co., Ltd.). The culture temperature is usually 20 to 40°C, preferably about 37°C. The culture time is usually 5 days to 3 weeks, preferably 1 to 2 weeks. Culture can be conducted usually in 5% $CO_2$ gas. The antibody titer in a culture supernatant of the hybridoma can be measured in the same manner as in the measurement of the anti-protein antibody titer in antiserum as described above.

(b) Purification of the monoclonal antibody

Separation and purification of the anti-protein monoclonal antibody can be performed according to the known method per se, for example, a method of separating and purifying an immunoglobulin, such as a salting-out method, an alcohol precipitation method, an isoelectric precipitation method, an electrophoresis method, an absorbing-desorbing method with an ion-exchanger (e.g., DEAE), an ultracentrifugation method, a gel filtration method, a specific purifying method for obtaining an antibody by collecting it exclusively on antigen-bound solid phase or on an active adsorbent such as protein A or protein G and then dissociating a bonding thereof to obtain the antibody.

[Preparation of a polyclonal antibody]

The polyclonal antibody of the present invention can be produced by the known method per se or its analogous method. For example, the polyclonal antibody can be produced by making an immunizing antigen (protein antigen)-carrier protein conjugate, then immunizing a warm-blooded animal therewith in the same manner as in the above method of producing the monoclonal antibody, collecting a material comprising an antibody against the protein of the present invention from the immunized animal, and separating and purifying the antibody.

For the immunizing antigen-carrier protein conjugate used for immunizing warm-blooded animals, the type of the carrier protein and the mixing ratio of the carrier to the hapten are not particularly limited insofar as the desired antibody can be efficiently produced by immunization with the antigen crosslinked via the hapten with the carrier, and for example, the conjugate is produced by coupling the hapten with bovine serum albumin, bovine cyloglobulin, hemocyanin or the like in a weight ratio of 1 to about from 0.1 to 20, preferably 1 to about from 1 to 5.

The hapten can be coupled with the carrier by use of various condensation agents such as glutaraldehyde, carbodiimide, maleimide-activated ester, and activated ester reagents containing thiol group and dithiopyridyl group.

The resulting condensation product is administered alone or together with a carrier and a diluent into warm-blooded animals at a site where the antibody can be produced. To enhance the ability of the animals upon administration to produce the antibody, complete Freund's adjuvant or incomplete Freund's adjuvant may be administered. Administration is conducted usually once every about 2 to 6 weeks and about 3 to 10 times in total.

The polyclonal antibody can be collected from blood, ascites, etc., preferably blood in the warm-blooded animals immunized by the method described above.

The polyclonal antibody titer in antiserum can be measured in the same manner as in the measurement of the antibody titer in antiserum as described above. Separation and purification of the polyclonal antibody can be carried out by a method of separating and purifying immunoglobulin, which is similar to the separation and purification of the monoclonal antibody as described above.

As the antisense DNA having a nucleotide sequence substantially complementary to a DNA or mRNA encoding the protein of the present invention or a partial peptide thereof, any antisense DNAs may be used as long as they are oligonucleotides or derivatives thereof having a nucleotide sequence substantially complementary to the whole or a part of the nucleotide sequence of a DNA or mRNA encoding the protein of the present invention or a partial peptide thereof and having an ability to suppress expression of said protein or a partial peptide thereof.

As the nucleotide sequence substantially complementary to the DNA or mRNA, for example, a nucleotide sequence having about 70% or more, preferably about 80% or more, more preferably about 90% or more, and most preferably about 95% or more homology with the whole or a part of a nucleotide sequence (that is, a complementary chain of said DNA or mRNA) complementary to said DNA or mRNA, etc., are exemplified. Particularly, antisense DNAs having about 70% or more, preferably about 80% or more, more preferably about 90% or more, and further preferably about 95% or more homology with the partial nucleotide sequence which codes, in the entire nucleotide sequence of the complementary chain of the DNA or mRNA of the present invention, for an N-terminal region (e.g., a nucleotide sequence in the vicinity of the initiation codon, etc.) of the protein of the present invention, etc. are preferable. These antisense DNAs can be produced using e.g., a known DNA synthesizer.

The protein of the present invention, a partial peptide thereof or a salt thereof (also referred to hereinafter as the protein of the present invention), the DNA encoding the protein of the present invention or a partial peptide thereof (also referred to hereinafter as the DNA of the present invention), the antibody against the protein of the present invention (also referred to hereinafter as the antibody of the present invention) and the antisense DNA can be used as a reagent in (1) a method for determining a binding protein to the protein of the present invention, (2) construction of a system for expressing a recombinant protein, (3) development of an assay system using the two-hybrid method and screening of candidates for pharmaceutical compounds, (4) practice of a drug design based on comparison with a structurally analogous ligand receptor and as (5) a reagent in formation of probes, PCR primers, etc. in genetic diagnosis etc., and further can be used as (6) a drug for gene therapy etc.

In particular, by acquisition of a binding protein to the protein of the present invention by using the two-hybrid method and further by a binding assay system using the protein of the present invention and an activin receptor or acquired other receptors, a promoter or an inhibitor in an information transmission system specific to warm-blooded animals such as human beings can be screened, and the promoter or the inhibitor can be used as an agent for preventing or treating various diseases, as described in detail below.

(1) A method for determining a binding protein to the protein of the present invention

The protein of the present invention is useful as a reagent for screening or determining a binding protein to the protein of the present invention.

That is, the present invention provides a method for determining a binding protein to the protein of the present invention, which comprises screening a binding protein interacting with the protein of the present invention.

Specifically, the method for determining a binding protein according to the present invention is a method for determining a protein or a salt thereof interacting with the protein of the present invention, which comprises introducing a vector having the protein of the present invention fused with a DNA binding region of a transcriptional factor on a host cell expression vector and a fusion library of a test protein with a transcription-activating region, into a host cell having a reporter gene maintaining said binding region of the transcriptional factor on a promoter, and measuring a change in the amount of the expressed reporter gene which is increased by binding of the two kinds of proteins.

The method for determining a binding protein according to the present invention comprises use of the two-hybrid method of detecting the binding protein by converting the interaction of the protein of the present invention with a test protein into the expression of a specific reporter gene.

The method for determining a binding protein according to the present invention is described below in more detail.

As the DNA encoding the protein of the present invention used in the method for determining a binding protein, any DNAs can be used insofar as they comprise a nucleotide sequence encoding a protein containing an amino acid sequence substantially identical to an amino acid sequence represented by SEQ ID NO:2 in the present invention or a nucleotide sequence encoding a partial peptide thereof. Further, the DNA may be a DNA isolated by a known method per se from a genomic DNA from warm-blooded animals (e.g., humans), a genomic DNA library from warm blooded animals (e.g., humans), a cDNA derived from tissues and cells in warm blooded animals (e.g., humans), a cDNA library derived from tissues and cells in warm-blooded animals (e.g., humans), a synthetic or semisynthetic DNA. The vector used in the library may be any of bacteriophage, plasmid, cosmid and phagemide, etc. Alternatively, the DNA can be isolated directly by the RT-PCR method using an mRNA fraction prepared from tissues and cells, or can also be produced by chemically producing partial nucleotide sequences respectively and ligating them.

As the test protein library screened, commercial cDNA libraries (e.g., MATCHMAKER cDNA produced by Clontech, etc.) derived from various organs of various animals, etc. are used.

As the vector expressing a fusion protein of the DNA-binding region with a test protein, pAS2-1, pGBT9, pKAD-09, pSD09, pEG202, pBTM116, etc. are used.

As the vector expressing a fusion protein of an activating region of a transcriptional factor with the protein, pGAD424, pACT2, pKT10Gal-VP, pJG4-5, pVP16, etc. are used.

As the transcriptional factor, GAL4, LexA, SRF, etc. are used.

As the reporter gene, β-galactosidase gene (LacZ), histidine gene (HIS3), luciferase gene, chloramphenicol acetyl transferase gene, etc. are used.

As the host cells, yeast (Saccharomyces cerevisiae), Escherichia coli, etc. are used, among which CG-1945, Y190, Y187, HF7c, SFY526, L40, EGY48, HIS/L1, 62L yeast strains, etc. are used.

Specifically, in the method for determining a binding protein to the present protein, first a plasmid in which a DNA fragment containing a nucleotide sequence encoding the present protein and a DNA fragment containing a nucleotide sequence encoding a DNA-binding region on a plasmid (e.g., pAS2-1) bound to the same frame, and a cDNA library expressed in the form of a fusion protein by ligating to a DNA fragment containing a nucleotide sequence encoding a transcription-activating region of a transcriptional factor (e.g., GAL4) on a plasmid (e.g., pACT2), can be formed according to a method described in, for example, Molecular Cloning, 2nd ed. (J. Sambrook et al., Cold Spring Harbor Lab. Press, 1989).

To introduce the two plasmids described above into host cells (e.g., Saccharomyces cerevisiae Y190), both the plasmids may be transformed simultaneously into the cells, or one plasmid may be first introduced followed by introducing the other plasmid, and for example, transformation can be carried out in a method described in, for example, Methods in Enzymology, 194, 182-187 (1991) or Proc. Natl. Acad. Sci. USA, 75, 1929 (1978).

Expression of a reporter gene in the transformant can be detected by conversion into the amount, emission, etc. of a coloring or fluorescent material formed by the reporter enzyme. More specifically, when the host cells are yeasts, expression of the reporter gene (e.g., β-galactosidase activity) can be detected by blue/white-coloring screening by use of the replica plate method or the filter method, preferably the filter method.

The colonies colored (e.g., blue) by the filter method are treated according to a method described in, for example, A Practical Approach (Bartel, P. L. et al., Oxford University Press, Oxford; 153-179, 1993a), whereby the positive clone can be separated as a single colony.

Recovery of the plasmid DNA from the single transformant thus separated is performed according to a method described in, for example, Gene, 57, 267 (1987), Bio Techniques, 14, 552 (1993), etc., and by determining the nucleotide sequence of said DNA, a receptor for the protein of the present invention can be determined.

Further, the binding protein to the protein of the present invention can also be determined by using a commercial kit, for example MATCHMAKER GAL4 Two-Hybrid Systems (Clontech) according to the manufacture's instructions.

(2) Agent for preventing or treating deficiency of the protein of the present invention

If the binding protein to the protein of the present invention is revealed in the method described in (1) above, the expression site of said binding protein and the action possessed by the protein of the present invention via said binding protein, etc., could be revealed. On the basis of these findings, the DNA encoding the protein of the present invention can be used as an agent for preventing or treating diseases occurring via said binding protein. Further, the protein of the present invention exhibits the binding activity to activin receptors, so it can be used as an agent for preventing or treating diseases occurring via the activin receptors.

For example, the gene for the protein of the present invention is expressed in heart, brain, liver, skeletal muscle, kidney, testis, lung and spleen, etc., and therefore, when there are those patients with diseases correlated with said binding protein or activin receptors, the action of the protein of the present invention in these organs in said patients can be sufficiently demonstrated by (A) administering the DNA encoding the protein of the present invention into the patients to express the protein, or (B) inserting the DNA encoding the protein of the present invention into cells of these organs, etc. to express the protein, followed by transplanting said cells in the patients. Accordingly, the DNA encoding the protein of the present invention can be used as a safe and low-toxic preventive or therapeutic agent for diseases occurring via the binding protein to the protein of the present invention.

To use the DNA of the present invention as the above therapeutic agent, said DNA can be conducted in a usual manner directly or after insertion thereof into a suitable vector such as retrovirus vector, adenovirus vector and adenovirus-associated virus vector, etc. For example, the DNA of the present invention can be used orally as tablets coated with sugar as necessary, as capsules, elixirs and microcapsules, or parenterally as an injection such as an aseptic solution with water or with other pharmaceutically acceptable solvents or suspension. For example, the DNA of the present invention can be formed by blending with physiologically acceptable carriers, flavors, excipients, vehicles, preservatives, stabilizers and binders, etc., into a unit dosage form required for the generally recognized preparation implementation. An amount of an active ingredient in these pharmaceutical preparations is such that a suitable volume in an indicated range can be obtained.

The additives which can be admixed with the tablets, capsules, etc. include, for example, binders such as gelatin, corn starch, tragacanth, gum arabic, excipients such as crystalline cellulose, swelling agents such as corn starch, gelatin and alginic acid, lubricants such as magnesium stearate, sweeteners such as sucrose, lactose and saccharine, and flavors such as peppermint, akamono oil and cherry. When one capsule is in a unit form, liquid carriers such as fats and oils can be contained in the materials described above. The aseptic composition for injection can be formulated according to conventional pharmaceutical manufacturing by dissolving or suspending the active material and naturally occurring vegetable oils such as sesame oil and coconut oil in vehicles such as injection water. The aqueous solution for injection includes physiological saline or an isotonic solution containing glucose and other supplementary agents (e.g., D-sorbitol, D-mannitol, sodium chloride, etc.), and may be used in combination with suitable solubilizers such as alcohols (e.g., ethanol etc.), polyalcohols (e.g., propylene glycol, polyethylene glycol, etc.) and nonionic surfactants (e.g., Polysorbate 80™, HCO-50, etc.). The oily solution includes sesame oil, soybean oil, etc., and may be used in combination with solubilizer such as benzyl benzoate, benzyl alcohol, etc.

Further, the above preventing or treating agent may be blended, for example, with the buffer (e.g., phosphate buffer, sodium acetate buffer, etc.), soothing agents (e.g., benzalkonium chloride, procaine hydrochloride, etc.), stabilizers (e.g., human serum albumin, polyethylene glycol, etc.), preservatives (e.g., benzyl alcohol, phenol, etc.), antioxidants, etc. Usually, the prepared injection is introduced into suitable ampoules. Since the thus obtained preparation is safe and low toxic, it can be administered, for example, to a mammal (e.g., rat, rabbit, sheep, pig, cow, cat, dog, monkey, human being, etc.). A dose of the DNA is different depending on symptom etc., and in the case of oral administration, the dose is usually about 0.1 to 100 mg, preferably about 1.0 to 50 mg and more preferably about 1.0 to 20 mg per day in an adult (60 kg). When parenterally administered, one time dose is different depending upon an administration subject, a subject organ, symptom and an administration method and, for example, in the form of an injection, it is advantageous to administer about 0.01 to 30 mg, preferably about 0.1 to 20 mg and more preferably about 0.1 to 10 mg per day through intravenous injection in an adult (60 kg). In the case of other animals, an amount calculated per 60 kg can be administered.

(3) A method of screening a compound inhibiting or promoting a binding of the protein of the present invention to its binding protein

By use of the protein of the present invention or a salt thereof, or by use of a protein competitive binding assay system using an expression system constructed for a recombinant binding protein, a compound (e.g., peptides, proteins, non-peptide compounds, synthetic compounds, fermentation products, etc.) or a salt thereof inhibiting or promoting a binding of the protein of the present invention to its binding protein can be screened. Further, by use of an expression system (two-hybrid method) for a reporter gene by interaction of the two kinds of proteins in a transformant into which a DNA encoding the protein of the present invention was introduced, a compound (e.g., peptides, proteins, non-peptide compounds, synthetic compounds, fermentation products, etc.) or a salt thereof inhibiting or promoting a binding of the protein of the present invention to its binding protein (e.g., activin receptor) can also be screened.

Such compounds include a compound (so-called an agonist against the protein of the present invention) having a cell-stimulating activity (e.g., the activity of promoting or inhibiting growth promotion and phosphorylation of intracellular proteins, etc.) via the binding protein and a compound (so-called an antagonist against the protein of the present invention) not having said cell-stimulating activity.

That is, the present invention provides:

A method of screening a compound or a salt thereof inhibiting or promoting a binding of the protein of the present invention to a binding protein to the protein of the present invention, which comprises comparing:

1) (i) the case where the protein of the present invention or a salt thereof is contacted with a binding protein to the protein of the present invention with (ii) the case where the protein of the present invention or a salt thereof and a test compound are contacted with a binding protein to the protein of the present invention, or
2) (i) a transformant into which a DNA encoding the protein of the present invention and a binding protein (e.g., activin receptor) to the protein of the present invention with (ii) said transformant brought into contact with a test compound.

The screening method of the present invention comprises comparing:

1) (i) the case where the protein of the present invention or a salt thereof is contacted with a binding protein to the protein of the present invention with (ii) the case where the protein of the present invention or a salt thereof and a test compound are contacted with a binding protein to the protein of the present invention, for example, by measuring the amount of the protein of the present invention or a salt thereof bound to a binding protein to the protein of the present invention, the cell-stimulating activity thereof, etc., or
2) (i) a transformant into which a DNA encoding the protein of the present invention and a binding protein (e.g., activin receptor) to the protein of the present invention with (ii) said transformant brought into contact with a test compound, for example, by measuring the degree of coloration, etc. indicative of the degree of expression of a reporter gene.

More specifically, the present invention provides:

(1) a method of screening a compound or a salt thereof inhibiting or promoting a binding of the protein of the present invention or a salt thereof to a binding protein to the protein of the present invention, which comprises comparing the case where the labeled protein of the present invention or a salt thereof is contacted with a binding protein to the present invention with the case where the labeled protein of the present invention or a salt thereof and a test compound are contacted with a binding protein to the protein of the present invention, by measuring the amount of the labeled protein or a salt thereof bound to the binding protein in both the cases,
(2) a method of screening a compound or a salt thereof inhibiting or promoting a binding of the protein of the present invention or a salt thereof to a binding protein to the protein of the present invention in the case where the binding protein to the present invention is a membrane-bound protein (e.g., membrane penetration receptor, channel, etc.), which comprises comparing the case where the labeled protein of the present invention or a salt thereof is contacted with cells containing the binding protein to the protein of the present invention or with a membrane fraction of said cells, with the case where the labeled protein of the present invention or a salt thereof and a test compound are contacted with cells containing the binding protein to the protein of the present invention or with a membrane fraction of said cells, by measuring the amount of the labeled protein of the present invention or a salt thereof bound to said cells or said membrane fraction in both the cases,
(3) a method of screening a compound or a salt thereof inhibiting or promoting a binding of the protein of the present invention or a salt thereof to a binding protein to the protein of the present invention, which comprises comparing the case where the labeled protein of the present invention or a salt thereof is contacted with the binding protein expressed in cells by culturing a transformant containing a DNA encoding the binding protein (e.g., an activin receptor) to the protein of the present invention, with the case where the labeled protein of the present invention or a salt thereof and a test compound are contacted with the binding protein expressed in cells by culturing a transformant containing a DNA encoding the binding protein to the protein of the present invention, by measuring the amount of the labeled protein of the present invention or a salt thereof bound to the binding protein in both the cases, and
(4) a method of screening a compound or a salt thereof inhibiting or promoting a binding of the protein of the present invention or a salt thereof to a binding protein to the protein of the present invention, which comprises comparing the expressions of a gene group whose expression is regulated by interaction between the protein of the present invention and the binding protein to the protein of the present invention, or physiological reaction (e.g., section of FSH, etc., when said binding protein is an activin receptor) in the host cells, in the case which a plasmid expressing a gene for the protein of the present invention and a plasmid expressing a gene for a binding protein (e.g., an activin receptor) to the protein of the present invention were introduced into host cells, and in the case which said host cells was brought into contact with a test compound.

Hereinafter, the screening method of the present invention is specifically described.
First, as the binding protein (e.g., an activin receptor) used in the screening method of the present invention, any proteins may be used as long as they contain said binding protein or a salt thereof, but extracts from organs in warm-blooded animals are preferable. However, since organs derived particularly from human beings are hardly obtainable, the binding protein or a salt thereof expressed in a large amount by genetic recombination technology is suitable for screening.
In order to prepare the binding protein, the aforementioned method is used, but preparation can be performed by expressing a DNA encoding said protein in a mammal cell or an insect cell. As a DNA fragment encoding a protein part of interest, a complementary DNA is usually used but is not necessarily limited to it. For example, a gene fragment or a synthetic DNA may be used. In order to introduce a DNA fragment encoding the binding protein into a host animal cell and express it effectively, it is preferable that the DNA fragment is integrated in downstream of polyhedron promoter from nuclear polyhedrosis virus (NPV) belonging to Baculovirus whose host is an insect, SV40-derived promoter, promoter of retrovirus, metallothionein promoter, human heat shock promoter, cytomegalovirus promoter, SR$\alpha$ promoter, etc. Investigation of an amount and quality of the expressed binding protein can be conducted by the known method per se. The investigation can be conducted by, for example, a method described in a reference [Nambi, P. et al., J. Biol. Chem., vol. 267, pp. 19555-19559, 1992].
Therefore, in the screening method of the present invention, a material comprising the binding protein or a salt thereof may be the binding protein or a salt thereof purified according to the known method per se, or a cell comprising said protein may be used, or when said protein is a membrane-bound protein, a membrane fraction of a cell containing the same may be used.
In the screening method of the present invention, when a cell comprising the binding protein is used, the cell may be fixed with glutaraldehyde, formalin, etc. The fixing method can be conducted according to the known method per se.

A cell comprising the binding protein refers to a host cell which expressed the binding protein and, as the host cell, Escherichia coli, Bacillus subtilis, yeast, insect cell, animal cell, etc. are exemplified.

A cell membrane fraction refers to a fraction in which a cell membrane obtained by the known method per se after disruption of a cell is contained in a large amount. As a method for disrupting a cell, there is a method of squeezing a cell with a Potter-Elvehjem type homogenizer, disruption with a Waring blender or Polytron (manufactured by Kinellmatica), disruption by sonication, and disruption by jetting a cell through a thin nozzle while pressurizing with a French press. For fractionating a cell membrane, a fractionating method by centrifugation force such as a fractionating centrifugation method and density gradient centrifugation method is mainly used. For example, a disrupted cell solution is centrifuged at a low speed (500 to 3000 rpm) in a short time (usually about 1 minute to 10 minutes), the supernatant is centrifuged at a higher speed (15000 to 30000 rpm) usually for 30 minutes to 2 hours, and the resulting precipitate is used as a membrane fraction. An expressed binding protein and membrane components such as cell-derived phospholipid and membrane protein are contained in a large amount in the membrane fraction.

An amount of a binding protein in a cell or a membrane fraction comprising the binding protein is preferably $10^2$ to $10^8$ molecules, suitably $10^5$ to $10^7$ molecules per cell. In addition, as an expressed amount is larger, the activity (specific activity) of binding to the protein of the present invention per a cellular extract or a membrane fraction is raised, and not only high sensitive screening system can be constructed but also a large amount of samples can be measured at the same lot.

In order to conduct the above methods (1) to (3) for screening a compound inhibiting binding of the protein of the present invention to a binding protein to the protein of the present invention, a suitable binding protein fraction and the labeled protein of the present invention are necessary. As the binding protein fraction, a natural binding protein fraction or a recombinant binding protein fraction having the equal activity thereto are desirable. Here, equal activity denotes an equal binding activity to the protein of the present invention, etc.

As the labeled protein of the present invention, the labeled protein of the present invention, a labeled compound analogous to the protein of the present invention, etc. is used. For example, the protein of the present invention labeled with [$^3$H], [$^{125}$I], [$^{14}$C], [$^{135}$S] or the like can be utilized.

Specifically, in order to conduct screening of a compound inhibiting binding of the protein of the present invention to a binding protein to the protein of the present invention, first, an authentic binding protein is prepared by suspending a cell or a membrane fraction of a cell comprising the binding protein in a buffer suitable for screening. Any buffers which do not inhibit binding of the protein of the present invention to a binding protein to the protein of the present invention, for example, phosphate buffer and Tris-HCl buffer, pH 4 to 10 (desirably pH 6 to 8), may be used. In addition, in order to decrease non-specific binding, surfactants such as CHAPS, Tween-80™ (Kao-Atlas Company), digitonin, and deoxycholate may be added to the buffer. Further, in order to suppress degradation of the binding protein and the protein of the present invention by a protease, a protease inhibitor such as PMSF, leupeptin, E-64 (manufactured by Peptide Laboratory) and pepstain may be added. The protein of the present invention labeled with a predetermined amount of (5000 to 500000 cpm) of the labeled protein of the present invention is added to 0.01 ml to 10 ml of the binding protein solution, and simultaneously $10^{-4}$ to $10^{-10}$ M test compound is allowed to be coexist. In order to know an amount of non-specific binding (NSB), a reaction tube to which the unlabeled protein of the present invention has been added in large excess is also prepared. The reaction is conducted at about 0 to 50°C, preferably about 4 to 37°C, for about 20 minutes to 24 hours, desirably about 30 minutes to 3 hours. After the reaction, the reaction solution is filtered with a glass fiber filtering paper and washed with a suitable amount of the same buffer, and the radioactivity remaining in the glass fiber filtering paper is measured by a scintillation counter or a γ-counter. Assuming that the count (B0 - NSB) obtained by subtracting the amount of non-specific binding (NSB) from the count (B0) in the absence of the antagonist is 100%, a test compound by which the amount of specific binding (B - NSB) becomes e.g., 50% or less can be selected as a candidate for a substance having an antagonistic inhibitory ability, while a test compound by which the amount of specific binding (B - NSB) becomes e.g., 150% or more can be selected as a candidate for a compound having a binding-promoting ability.

To practice the above-mentioned method (4) for screening a compound inhibiting or promoting a binding of the protein to a binding protein to the protein of the present invention, expression of a gene group whose expression is regulated by interaction between the protein of the present invention and a binding protein to the protein of the present invention, or physiological reactions (e.g., secretion of FSH, etc. when the binding protein is an activin receptor) in a host cell to which a plasmid expressing a gene for the protein of the present invention and a plasmid expressing a gene for a binding protein (e.g., an activin receptor) to the protein of the present invention were introduced can be measured by a known method. Specifically, when the host cell is yeast, a transformant transformed by introducing, to a yeast cell, a plasmid expressing a fusion protein between the protein of the present invention and a DNA-binding region of a transcription-regulating factor and a plasmid expressing a fusion protein between a binding protein (e.g., activin receptor) to the protein of the present invention and an activating region of a transcription-regulating factor is created by the same method as described above. For screening, this transformant is cultured at 30°C for 2 to 4 days on an agar medium containing $10^{-4}$ to $10^{-10}$ M test compound. As the agar medium, a tryptophan/leucine/histidine-deficient SD medium, a tryptophan/leucine-deficient SD medium, etc. are used. Thereafter, the filter method or the like is used in order to detect expression of the reporter gene as coloration of colonies by β-galactosidase activity. For this detection, a Whatman #5 filter or VWR grade 410 filter moistened with buffer Z/X-gal (Clontech) is placed on a filter to which the transformant adhered, and incubated at 30°C for 30 minutes to 8 hours, and then the coloration of the colonies on the filter is compared with the coloration of a control transformant not containing a test compound. A test compound added to the culture medium of colonies showing darker coloration than that of the control can be selected as a candidate for a compound having an ability to promote binding, while a test compound added to the culture medium of colonies showing lighter coloration than that of the control can be selected as a candidate for a compound having an ability to inhibit binding. Further, expression of the reporter gene can also be quantified as β-galactosidase activity by measuring the amount of o-nitrophenol formed by decomposition of the substrate. First, the transformant is cultured under shaking at 30°C for 8 to 24 hours in a liquid medium containing $10^{-4}$ to $10^{-10}$ M test compound. As the liquid medium, a tryptophan/leucine/histidine-deficient SD medium, a tryptophan/leucine-deficient SD medium, etc. are used. Preferably, after overnight culture, an aliquot of the culture liquid is inoculated into YPD medium and cultured under shaking at 30°C for 3 to 5 hours during which the $OD_{600}$ of 0.5 is increased to 1.0. To a mixture of the resulting precipitate obtained by centrifugation of an aliquot of this culture liquid in buffer Z/β-mercaptoethanol is added an o-nitrophenyl galactoside solution (Sigma), and the mixture is reacted. The reaction is carried out at 0 to 50°C for 3 minutes to 24 hours, desirably at 30°C for 30 minutes to 15 hours, and the absorbance ($OD_{420}$) of the thus yellowed supernatant at 420 nm is measured. From the absorbance ($OD_{420}$) thus obtained, the β-galactosidase activity is calculated as Miller unit by use of the equation below.

In the case where upon adding a test compound to the medium, the β-galactosidase activity is increased by about 10% or more, preferably about 20% or more, more preferably about 30% or more and most preferably about 50% or more, said test compound can be selected as a candidate for a compound having an ability to promote binding between the protein of the present invention and a binding protein to the protein of the present invention. On the other hand, in the case where upon adding a test compound to the medium, the β-galactosidase activity is decreased by about 10% or more, preferably about 20% or more, more preferably 30% or more and most preferably about 50% or more, said test compound can be selected as a candidate for a compound having an ability to inhibit binding between the protein of the present invention and a binding protein to the protein of the present invention.

To perform screening by measuring the expression activity of the reporter gene, a DNA encoding the binding protein (e.g., activin receptor) to the protein of the present invention is necessary. As the DNA encoding the binding protein to the protein of the present invention, a DNA comprising said DNA, a DNA hybridizing therewith under high stringent conditions, etc. are desirable.

As the test compound, for example, peptides, proteins, non-peptide compounds, synthetic compounds, fermentation products, cellular extracts, plant extracts, animal tissue extracts, etc. are exemplified, and these compounds may be novel compounds or known compounds.

A kit for screening a compound or a salt thereof inhibiting or promoting a binding of the protein of the present invention to a binding protein to the protein of the present invention comprises the protein of the present invention or a salt thereof, a partial peptide thereof or a salt thereof, cells containing the binding protein to the protein of the present invention, a fraction extracted from cells containing the binding protein to the protein of the present invention, or a transformant into which a plasmid expressing a fusion protein between the protein of the present invention and a DNA-binding region of a transcription-regulating factor and a plasmid expressing a fusion protein between the binding protein to the protein of the present invention and an activating region of a transcription-regulating factor were introduced.

As the screening kit of the present invention, for example the following is exemplified.

1. Reagents for screening

(1) Transformant

Yeast Y190 strain transformed by introducing a plasmid expressing a fusion protein between the protein of the present invention and a DNA-binding region of a transcription-regulating factor and a plasmid expressing a fusion protein between an activin IIA receptor protein and an activating region of a transcription-regulating factor.

(2) Liquid culture medium

Tryptophan/leucine/histidine-deficient SD medium: An aqueous solution of a yeast nitrogen source (Difco) is sterilized in an autoclave, and then a drop-out solution consisting of L-isoleucine, L-valine, L-adenine hemisulfate, L-arginine hydrochloride, L-lysine hydrochloride, L-methionine, L-phenyl alanine, L-threonine, L-tyrosine and L-uracil, which was sterilized in an autoclave and stored at 4°C, is added thereto. Further, 40% dextrose/stock solution (Sigma) sterilized by filtration with a filter is added thereto at a concentration of 2%, which may be stored at 4°C or prepared when the medium is used.

YPD medium: Difco peptone and an aqueous solution of a yeast extract are sterilized in an autoclave, and 40% dextrose sterilized by filtration with a filter is added thereto at a final concentration of 2%, which may be stored at 4°C or prepared when the medium is used.

(3) Buffer

Buffer Z: A buffer containing $Na_2HPO_4 \cdot 7H_2O$, $NaH_2PO_4 \cdot H_2O$, KCl, $MgSO_4 \cdot 7H_2O$ is adjusted to pH 7 or thereabout and sterilized in an autoclave, which may be stored at 4°C or prepared when the buffer is used.

Buffer Z/β-mercaptoethanol: A mixture of 100 parts of buffer Z and 0.27 part of β-mercaptoethanol.

(4) Substrate for β-galactosidase

An o-nitrophenyl galactoside solution (Sigma) is dissolved in buffer Z and adjusted to a concentration of 4 mg/ml, and this substrate is prepared when it is used.

(5) Reaction termination solution

1 M sodium carbonate solution stored at 4°C is used, and this solution may be prepared when it is used.

2. Measurement method

(1) A yeast transformant is cultured under shaking at 30°C overnight in 1 ml tryptophan/leucine-deficient SD medium containing 5 μl of $10^{-3}$ to $10^{-10}$ M test compound solution.

(2) 0.4 ml of the culture liquid is added to 1.6 ml YPD medium and cultured under shaking at 30°C for 3 to 5 hours during which the $OD_{600}$ of 0.5 is increased to 1.0.

(3) 0.3 ml of the culture liquid is centrifuged at 14000 rpm for 30 seconds, and the resulting residue are suspended in 0.3 ml buffer Z and centrifuged again, and the resulting precipitates are suspended in 0.1 ml buffer Z.

(4) After the suspension is frozen once in liquid nitrogen, it is melted at 37°C for 30 seconds to 1 minute. 0.7 ml mixture of buffer Z/β-mercaptoethanol and 0.16 ml o-nitrophenyl galactoside solution are added thereto, and the mixture is incubated at 30°C until the solution turns yellow, and thereafter, 0.4 ml of 1 M sodium carbonate solution is added thereto.

(5) The mixture is centrifuged at 14000 rpm for 10 minutes, and the absorbance of its supernatant at 420 nm is measured, and the β-galactosidase activity is calculated as Miller unit by use of Equation 1 below.

[Equation 1]

$$\beta\text{-Galactosidase activity} = 1000 \times OD_{420}/(t \times V \times OD_{600})$$

$OD_{420}$: Absorbance at 420 nm

t: Reaction time (min)

V: The volume of a suspension of the transformant in buffer Z, used in the reaction × the degree of dilution

A compound or a salt thereof obtained by use of the screening method or the screening kit according to the present invention is either a compound inhibiting binding between the present protein (particularly the protein which has PDZ domain, is expressed particularly in the heart, the brain, the liver, the skeletal muscle, the kidney, the testis, the lung or the spleen) and a binding protein (e.g., an activin receptor) to the protein of the present invention, or a compound promoting said binding (hereinafter, a promoter).

The compound inhibiting binding of the protein of the present invention to a binding protein (e.g., an activin receptor) to the protein of the present invention includes (1) a compound or a salt thereof (so-called an agonist) which binds to a binding protein to the protein of the present invention thereby inhibiting binding between the protein of the present invention and the binding protein to the protein of the present invention and which in itself has a cell-stimulating activity via the binding protein, (2) a compound or a salt thereof (so-called an antagonist) which binds to a binding protein to the protein of the present invention thereby inhibiting binding between the protein of the present invention and the binding protein to the protein of the present invention but which in itself does not have a cell-stimulating activity via the binding protein, and (3) a compound (referred to hereinafter as an inhibitor) or a salt thereof which inhibits binding between the protein of the present invention and a binding protein to the present invention without binding to the binding protein to the protein of the present invention.

Whether the binding between the compound or a salt thereof obtained by using the screening method or the screening kit according to the present invention and the binding protein to the protein of the present invention is present or not can be confirmed according to the above method of measuring the binding activity.

The cell-stimulating activity via the binding protein can be measured according to the known method per se or its analogous method.

As the compound obtained by using the screening method or the screening kit according to the present invention, peptides, proteins, non-peptide compounds, synthetic compounds, fermentation products, etc. are exemplified, and these compounds may be novel compounds or known compounds.

The agonist and the promoter have the same action as the physiological activity possessed by the protein of the present invention or have the action of enhancing its physiological activity, so that depending on the activity of said protein, they are useful in a safe and low-toxic pharmaceutical composition, particularly as an agent for preventing or treating diseases (for example, nerve cell abnormalities or cerebral diseases, such as Alzheimer's disease, Parkinson's disease, epilepsy, Huntington's chorea) correlated with said binding protein or activin receptors in the heart, the brain, the liver, the skeletal muscle, the kidney, the testis, the lung, the spleen, etc.

On the other hand, since the antagonist or the inhibitor can inhibit the physiological activity possessed by the protein of the present invention, it can be used in a safe and low-toxic pharmaceutical composition inhibiting the activity of said protein, particularly as an agent for preventing or treating diseases (for example, nerve cell abnormalities or cerebral diseases, such as Alzheimer's disease, Parkinson's disease, epilepsy, Huntington's chorea) correlated with said binding protein or activin receptors in the heart, the brain, the liver, the skeletal muscle, the kidney, the testis, the lung, the spleen.

When the compound or a salt thereof obtained by using the screening method or the screening kit according to the present invention is used as the aforementioned pharmaceutical composition, a conventional means can be used. For example, said compound or a salt thereof can be used orally as tablets coated with sugar as necessary, capsules, elixirs and microcapsules, or parenterally an injection such as an aseptic solution or suspension with water or with other pharmaceutically acceptable solutions. For example, said compound or a salt thereof can be formed by blending with physiologically acceptable carriers, flavors, excipients, vehicles, preservatives, stabilizers, binders, etc. into a unit dosage form required for the generally recognized preparation implementation. An amount of the active ingredient: in these pharmaceutical preparations is such that a suitable volume in an indicated range can be obtained.

The additives which can be admixed into the tablets, capsules, etc. include, for example, binders such as gelatin, corn starch, tragacanth and gum arabic, excipients such as crystalline cellulose, swelling agents such as corn starch, gelatin and alginic acid, lubricants such as magnesium stearate, sweeteners such as sucrose, lactose and saccharine, and flavors such as peppermint, akamono oil and cherry. When one capsule is in a unit form, liquid carriers such as fats and oils can be contained in the materials described above. The aseptic composition for injection can be formulated according to conventional pharmaceutical manufacturing by dissolving or suspending the active material and naturally occurring vegetable oils such as sesame oil and coconut oil in vehicles such as injection water.

The aqueous solution for injection includes, for example, physiological saline or an isotonic solution containing glucose and other supplementary agents (e.g., D-sorbitol, D-mannitol, sodium chloride, etc.), and may be used in combination with suitable solubilizers such as alcohols (e.g., ethanol), polyalcohols (e.g., propylene glycol and polyethylene glycol), nonionic surfactants (e.g., Polysorbate 80™ and HCO-50), etc. The oily solution includes, for example, sesame oil, soybean oil, etc., and may be used in combination with solubilizer such as benzyl benzoate, benzyl alcohol, etc. Further, the above preventing or treating agent may be blended, for example, with the buffer (e.g., phosphate buffer and sodium acetate buffer), soothing agents (e.g., benzalkonium chloride, procaine hydrochloride, etc.), stabilizers (e.g., human serum albumin, polyethylene glycol, etc.), preservatives (e.g., benzyl alcohol, phenol, etc.), antioxidants, etc. Usually, the prepared injection is introduced into suitable ampoules. Since the thus obtained preparation is safe and low toxic, it can be administered, for example, to a warm-blooded mammal (e.g., rat, rabbit, sheep, pig, cow, cat, dog, monkey, human being, etc.).

A dose of said compound or a salt thereof is different depending on symptom etc., and in the case of oral administration, the dose is usually about 0.1 to 100 mg, preferably about 1.0 to 50 mg and more preferably about 1.0 to 20 mg per day in an adult (60 kg). When parenterally administered, one time dose is different depending upon an administration subject, a subject organ, symptom, an administration method, etc. and, for example, in the form of an injection, it is advantageous to administer about 0.01 to 30 mg, preferably about 0.1 to 20 mg and more preferably about 0.1 to 10 mg per day through intravenous injection in an adult (60 kg). In the case of animals other than human beings, an amount calculated per 60 kg can be administered.

(4) Quantification of the protein of the present invention, a partial peptide or a salt thereof

The antibody against the protein of the present invention can specifically recognize the protein etc. of the present invention so that it can be used, for example, in quantification of the protein etc. of the present invention in a test sample, particularly in quantification thereof by sandwich immunoassays.

That is, the present invention provides:

(i) a method of quantifying the protein etc. of the present invention in a test solution, which comprises allowing a test solution and the labeled protein etc. of the present invention to react competitively with an antibody reacting with the protein etc. of the present invention and determining the ratio of the labeled protein etc. of the present invention bound to said antibody; and

(ii) a method of quantifying the protein etc. of the present invention in a test solution, which comprises allowing a test solution to react with the antibody of the present invention insolubilized on a carrier and the labeled antibody of the present invention simultaneously or successively and then measuring the activity of the labeling agent on the insolubilizing carrier, wherein one of the two antibodies is an antibody recognizing an N-terminal region or a C-terminal region of the protein etc. of the present invention, and the other antibody is an antibody reacting with an amino acid sequence of SEQ ID NO:2.

The monoclonal antibody recognizing the protein etc. of the present invention (hereinafter, also referred to as the anti-protein antibody) can be used not only for quantifying the protein etc. of the present invention but also for detection thereof by tissue staining etc. For these purposes, the antibody molecule itself may be used, or an $F(ab')^2$, Fab' or Fab fraction of the antibody molecule may be used.

The method of quantifying the protein etc. of the present invention using the antibody of the present invention is not particularly limited as long as the method comprises detecting the amount of the antibody, the antigen or an antigen-antibody conjugate, corresponding to the amount of the antigen (e.g., the amount of the protein of the present invention) in a test solution, by chemical or physical means and calculating it on the basis of a standard curve prepared using standard solutions containing known amounts of the antigen. For example, nephelometry, the competitive method, the immunometric method and the sandwich method are preferably used, but the sandwich method described later is particularly preferably used in respect of sensitivity and specificity.

The labeling agent used in the measurement method using a labeling substance includes, for example, a radioisotope, enzyme, fluorescent material and luminescent material. The radioisotope includes, for example, [$^{125}I$], [$^{131}I$], [$^{3}H$], [$^{14}C$], etc.; the enzyme described above is preferably a stable enzyme with high specific activity, which includes, for example, β-galactosidase, β-glucosidase, alkali phosphatase, peroxidase, malate dehydrogenase, etc.; the fluorescent material includes, for example, fluorescamine and fluorescein isocyanate; the luminescent material includes luminol, luminol derivatives, luciferin and lucigenin. Further, a biotin-avidin system can also be used for binding the antibody or antigen to the labeling agent.

The antigen or antibody may be insolubilized by physical adsorption or via chemical bonding used conventionally for insolubilization or immobilization of proteins, enzymes, etc. The carrier includes insoluble polysaccharides such as agarose, dextran and cellulose, synthetic resin such as polystyrene, polyacrylamide and silicon, and glass, etc.

In the sandwich method, a test sample is allowed to react with the insolubilized anti-protein antibody (primary reaction), then the labeled anti-protein antibody is allowed to react therewith (secondary reaction), and the activity of the labeling agent on the insolubilizing carrier is measured, whereby the protein of the present invention in the test sample can be quantitatively determined. The primary and secondary reactions may be carried out in the reverse order, simultaneously, or separately after a predetermined time. The labeling agent and the method of insolubilization may be in accordance with those described above. The antibody used as the solid-phase antibody or as the labeling antibody in immunoassays by the sandwich method may not necessarily be one kind of antibody and may use a mixture of two or more kinds of antibodies for the purpose of improving measurement sensitivity etc.

In the method of measuring the protein etc. of the present invention by the sandwich method according to the present invention, the anti-protein antibodies used in the primary and secondary reactions are preferably those antibodies which bind the protein etc. of the present invention at different sites. That is, when the antibody used in, for example, the secondary reaction recognizes a C-terminal region or a N-terminal region of the protein etc. of the present invention, the other antibody used in the primary reaction recognizes preferably an amino acid sequence represented by SEQ ID NO:2.

The antibody against the protein etc. of the present invention can be used not only in the sandwich method but also in other measurement systems such as competitive method, immunometric method, nephelometry, etc.

In the competitive method, an antigen in a test sample and its labeled antigen are allowed to react competitively with the antibody, then the unreacted labeled antigen (F) and the labeled antibody (B) bound to the antibody are separated from each other (B/F separation), and the amount of either labeled B or F is determined to quantify the amount of the antigen in the sample. This reaction method employs either a liquid-phase method in which a soluble antibody is used as the antibody and polyethylene glycol and a second antibody against the above antibody are used in B/F separation, or a solid-phase method in which a solid-phase antibody is used as the first antibody, or a soluble antibody is used as the first antibody while a solid-phase antibody is used as the second antibody.

In the immunometric method, the antigen in a test solution and the solid-phase antigen are allowed to react competitively with a predetermined amount of the labeled antibody, followed by separating the solid phase from the liquid phase, or alternatively the antigen in a test solution is allowed to react with an excess amount of the labeled antibody, and then the solid-phase antigen is added thereto to permit the unreacted labeled antibody to be bound to the solid phase, followed by separating the solid phase from the liquid phase. Then, the amount of the label in either phase is measured to quantify the amount of the antigen in the test solution.

Further, in nephelometry, the amount of insoluble precipitates in gel or solution, formed by antigen-antibody reaction, is measured. Laser nephelometry using laser scattering can be applied preferably to the case where precipitates are obtained in a small amount because of a very small amount of antigen in a test solution.

For application of these immunoassays to the quantification method of the present invention, it is not necessary to establish special conditions, procedures, etc. A measurement system for the protein etc. of the present invention can be established in an ordinary manner by those skilled in the art by modifying the conventional conditions and procedures. The details of these general technical means can be referred to in general remarks, books, etc. [for example, reference can be made of "Radioimmunoassays" edited by Hirhoshi Irie (published by Kodansha in 1974), "Radioimmunoassays" (2nd edition) edited by Hiroshi Irie (published by Kodansha in 1979), "Enzyme Immunoassays" edited by Eiji Ishikawa et al. (published by Igakushoin in 1978), "Enzyme Immunoassays" (2nd edition) edited by Eiji Ishikawa et al. (published by Igakushoin in 1982), "Enzyme Immunoassays" (3rd edition) edited by Eiji Ishikawa et al. (published by Igakushoin in 1987), Methods in Enzymology, Vol. 70 (Immunochemical Techniques (Part A)), Methods in Enzymology, Vol. 73 (Immunochemical Techniques (Part B)), Methods in Enzymology, Vol. 74 (Immunochemical Techniques (Part C)), Methods in Enzymology, Vol. 84 (Immunochemical Techniques (Selected Immunoassays (Part D)), and Methods in Enzymology, Vol. 92 (Immunochemical Techniques (Monoclonal Antibodies and General Immunoassay Methods (Part E)), Methods in Enzymology, Vol. 121 (Immunochemical Techniques (Hybridoma Technology and Monoclonal Antibodies (Part I)) (which are published by Academic Press Ltd.).

By using the antibody of the present invention in the manner as described above, the protein etc. of the present invention can be quantified with good sensitivity.

Further, by quantifying the concentration of the protein etc. of the present invention by means of the antibody against the protein etc. of the present invention, for example, diagnosis of diseases correlated with the protein etc. of the present invention can be performed.

Further, the antibody of the present invention can be used to detect the protein etc. of the present invention present in humor and tissues. In addition, the antibody of the present invention can be used for preparation of an antibody column used in purification of the protein etc. of the present invention, for detection of the protein etc. of the present invention in each fraction during purification, for analysis of the behavior of the protein etc. of the present invention in cells examined, etc.

(5) Genetic diagnostic agent

Since the DNA of the present invention can be used for example as a probe to detect abnormalities in a gene encoding the protein of the present invention or a partial peptide thereof in a human being or a warm-blooded animal (e.g., rat, mouse, guinea pig, rabbit, bird, sheep, pig, cow, horse, cat, dog, monkey, chimpanzee, etc.), the DNA of the present invention is useful as a genetic diagnostic agent for e.g., mutation of said DNA, abnormal accumulation or abnormal reduction of the mRNA, etc.

This genetic diagnosis using the DNA of the present invention can be carried out by techniques known per se, for example, Northern hybridization, the PCR-SSCP method (Genomics, Vol. 5, pp. 874-879 (1989) and Proceedings of the National Academy of Sciences of the United States of America, Vol. 86, pp. 2766-2770 (1989)), etc.

(6) DNA containing an antisense DNA

An antisense DNA capable of binding complimentarily to the DNA or mRNA encoding the protein etc. of the present invention to suppress transcription or translation of the DNA or mRNA can suppress abnormal expression of a gene encoding the protein etc. of the present invention. Therefore, the antisense DNA can be used as e.g., a therapeutic or preventive agent for diseases attributable to abnormal expression of a gene encoding the protein etc. of the present invention.

When the antisense DNA is used as the above therapeutic or preventive agent, it can be produced in the same manner as for the aforementioned pharmaceutical preparation containing the DNA of the present invention. For example, said antisense DNA can be administered into a human being or a warm-blooded animal in a usual manner directly or after insertion thereof into a suitable vector such as retrovirus vector, adenovirus vector and adenovirus-associated virus vector. The antisense DNA is formed alone or along with physiologically acceptable carriers such as a supplementary agent for promoting ingestion, into a pharmaceutical preparation which can be administered via a gene gun or a catheter such as hydrogel catheter.

Further, said antisense DNA can also be used as a diagnostic oligonucleotide probe for examining the presence or expression of the DNA of the present invention in tissues or cells.

(7) Creation of DNA-transferred animals

The present invention provides non-human mammals having an extraneous DNA encoding the protein etc. of the present invention (referred to hereinafter as the extraneous DNA of the present invention) or a variant DNA thereof (also referred to hereinafter as the extraneous variant DNA of the present invention).

That is, the present invention provides:

(i) non-human mammals having the extraneous DNA of the present invention or a variant DNA thereof;
(ii) the non-human mammals according to item (i), wherein the non-human mammals are rodents;
(iii) the non-human mammals according to item (ii), wherein the rodents are mice;
(iv) the non-human mammals according to item (ii), wherein the rodents are rats; and
(v) a recombinant vector comprising the extraneous DNA of the present invention or a variant DNA thereof and capable of expression in mammals.

The non-human mammals having the extraneous DNA of the present invention or its variant DNA (hereinafter, referred to as the DNA-transferred animals of the present invention) can be produced by transferring the intended DNA to embryonic cells such as unfertilized cells, fertilized cells, sperms and their initial cells, preferably at the stage of embryonic development in the development of non-human mammals (more preferably at the stage of single cells or fertilized egg cells and generally before the 8-cell stage) by the calcium phosphate method, electric pulse method, lipofection method, aggregation method, microinjection method, particle gun method, DEAE-dextran method or retrovirus method. By the method of transferring the DNA, the intended extraneous DNA of the present invention can be transferred to somatic cells, organs in a living body, tissue cells, etc. and utilized in cell culture and tissue culture, and these cells can also be fused with the above-mentioned embryonic cells by the cell fusion method known per se in order to produce the DNA-transferred animals of the present invention.

The non-human mammals used include, for example, rat, mouse, guinea pig, hamster, rabbit, sheep, pig, cow, cat, dog, etc. In particular, rodents, particularly mice (for example, C57BL/6 strain, DBA2 strain, etc. as pure strain, and $B6C3F_1$ strain, $BDF_1$ strain, $B6D2F_1$ strain, BALB/c strain, ICR strain, etc. as crossed strain), rats (for example, Wister, SD, etc.), etc. are preferable in formation of a morbid animal model system because of their easy breeding with a relatively short period of developments and biological cycle.

With respect to the recombinant vector capable of expression in mammals, the "mammals" include human beings in addition to the non-human mammals described above.

The extraneous DNA of the present invention is not the DNA of the present invention inherent in non-human mammals but the DNA encoding the protein of the present invention, which was once isolated and extracted from mammals.

The variant DNA of the present invention includes a DNA derived from the original DNA of the present invention by varying (for example, mutating etc.) the nucleotide sequence thereof, specifically by adding or deleting some bases or by substituting some bases with other bases, and the variant DNA also includes an abnormal DNA.

The abnormal DNA refers to a DNA comprising a gene expressing the abnormal protein of the present invention, and for example, a DNA comprising a gene expressing a protein suppressing the functions of the normal protein of the present invention etc. is used.

The extraneous DNA of the present invention may be derived from mammals of either the same species as or a different species from that of the intended animals. For transferring the DNA encoding the protein of the present invention to the intended animals, the DNA is generally advantageously used as a DNA construct having the DNA bound to a region downstream from a promoter capable of expressing the DNA in animal cells. For example, in the case where the DNA of the present invention is to be transferred, a DNA construct (e.g., a vector etc.) having the DNA of the present invention bound to a region downstream from a promoter permitting expression of a DNA having high homology with the DNA of the present invention and derived from various mammals (e.g., rabbit, dog, cat, guinea pig, hamster, rat, mouse, etc.) carrying the DNA of the present invention is microinjected into fertilized eggs of the intended animals, such as mouse fertilized eggs, whereby DNA-transferred mammals highly expressing the DNA of the present invention can be produced.

The vector carrying said construct includes plasmids derived from E. coli, Bacillus subtilis or yeast, bacteriophage such as λ-phage, retrovirus such as Maloney leukemia virus, and animal viruses such as vaccinia virus and Baculovirus. In particular, the plasmids derived from E. coli or yeast are preferably used.

The promoter for regulating expression of the DNA includes, for example, promoters derived from viruses (e. g., simian virus, cytomegalovirus, Maloney leukemia virus, JC virus, breast cancer virus, poliovirus, etc.), promoters derived from various mammals (human being, rabbit, dog, cat, guinea pig, hamster, rat, mouse, etc.), for example, promoters for albumin, insulin II, Uroplakin II, elastase, erythropoietin, endoserine, muscular creatine kinase, glia fibrous acidic protein, glutathione S-transferase, platelet-derived growth factor β, keratin K1, K10 and K14, collagen I and II type, cyclic AMP-dependent kinase βI subunit, dystrophin, tartaric acid-resistant alkali phosphatase, atrial natriuretic factor, endothelial receptor tyrosine kinase (abbreviated generally to Tie 2), sodium potassium adenosine triphosphatase (Na, K-ATPase), neurofilament light chain, metallothionein I and IIA, metalloproteinase 1 tissue inhibitor, MHC class I antigen (H-2L), H-ras, renin, dopamine β-hydroxylase, thyroid peroxidase, polypeptide chain-extending factor 1α (EF-1α), β-actin, α- and β-myosin heavy chains, myosin light chains 1 and 2, myelin basic protein, thyroglobulin, Thy-1, immunoglobulin, H chain variable region (VNP), serum amyloid P component, myoglobin, troponin C, smooth muscular α-actin, preproenkephalin A and vasopressin, among which promoters such as cytomegalovirus promoter, human polypeptide-extending factor 1α (EF-1α) promoter, human and chicken β-actin promoters can be preferably used because of their ability to highly express the DNA in the whole body.

Preferably, the vector has a sequence (generally called a terminator) for terminating transcription of the intended mRNA in the DNA-transferred mammals, and, for example, the sequence of each DNA derived from viruses or various mammals, preferably SV40 terminator from simian virus, can be used.

Further, a splicing signal for each DNA, an enhancer region, a part of introns from eucaryotic DNA, etc. can also be linked depending on the object to a 5'-upstream region from the promoter, to a region between the promoter and the translating region, or to a 3'-downstream region from the translating region.

The translating region for the normal protein of the present invention can be obtained in the known method per se as the whole or a part of genomic DNA derived from the liver, kidney, thyroid cells, and fibroblasts derived from various mammals (e.g., rabbit, dog, cat, guinea pig, hamster, rat, mouse, human being, etc.) and genomic DNA from a variety of commercial genomic DNA libraries or by use of a complementary DNA as the starting material prepared in the known method per se from RNA derived from the liver, kidney, thyroid cells, and fibroblasts. Further, the extraneous abnormal DNA can be obtained from the above cells or tissues with diseases attributable to a variation in the protein of the present invention. In addition, a variant translating region can be produced by varying a translating region of the normal protein obtained from the above cells or tissues by means of a point mutagenesis method.

The translating region can be constructed as a DNA construct capable of expression in the DNA-transferred animals by conventional DNA engineering means for linking it to a downstream region from the promoter or if desired to an upstream region from the transcription termination site.

The extraneous DNA of the present invention is transferred to cells at the stage of fertilized egg cells so as to secure the presence of the DNA in all embryonic cells and somatic cells of the intended mammals. The presence of the extraneous DNA of the present invention in the embryonic cells in the produced animals after transfer of the DNA means that the extraneous DNA of the present invention is maintained in all embryonic cells and somatic cells in the offspring of the produced animals. The offspring of the produced animals, which has inherited the extraneous DNA of the present invention, has the extraneous DNA of the present invention in all embryonic cells and somatic cells thereof.

After it is confirmed that the non-human mammals having the extraneous normal DNA of the present invention transferred to them maintain the DNA stably through crossbreeding, they can be bred generation after generation as animals carrying said DNA under normal breeding environments.

The extraneous DNA of the present invention is transferred to cells at the stage of fertilized egg cells so as to secure the presence of the DNA in excess in all embryonic cells and somatic cells in the intended mammals. The presence of the extraneous DNA of the present invention in excess in the embryonic cells in the produced animals after transfer of the DNA means that the extraneous DNA of the present invention is maintained in excess in all embryonic cells and somatic cells in the offspring of the produced animals. The offspring of the produced animals, which has inherited the extraneous DNA of the present invention, has the extraneous DNA of the present invention in excess in all embryonic cells and somatic cells thereof. Homozygote animals having the introduced DNA in both homogenous chromosomes are obtained, and these male and female animals are crossbred to produce the offspring having said DNA in excess and capable of breeding generation after generation.

The non-human mammals having the normal DNA of the present invention can be used as a morbid animal model since the normal DNA of the present invention is highly expressed in these animals to promote the functions of endogenous normal DNA, resulting in the onset of functional exasperation caused by the protein of the present invention. For example, the normal DNA-transferred animals of the present invention can be used to elucidate the mechanism of the morbid functional exasperation caused by the protein of the present invention or diseases correlated with the protein of the present invention or to examine the method of treating these diseases.

Further, the mammals having the extraneous normal DNA of the present invention transferred thereto can also be used in a test for screening a therapeutic agent against diseases correlated with the protein of the present invention because these animals have the symptom of an increase in the protein of the present invention.

On the other hand, the non-human mammals having the extraneous abnormal DNA of the present invention transferred thereto are confirmed to maintain the extraneous DNA stably through crossbreeding, and they can be then bred generation after generation as animals carrying said DNA under normal breeding environments. Further, the desired extraneous DNA can be used as a starting material by integrating it in the plasmid described above. The DNA construct having the promoter can be produced in usual DNA engineering means. The abnormal DNA of the present invention is transferred to cells at the stage of fertilized egg cells so as to secure the presence of the DNA in all embryonic cells and somatic cells of the intended mammals. The presence of the abnormal DNA of the present invention in the embryonic cells in the produced animals after transfer of the DNA means that the abnormal DNA of the present invention is maintained in all embryonic cells and somatic cells in the offspring of the produced animals. The offspring of the produced animals, which has inherited the DNA, has the abnormal DNA of the present invention in all embryonic cells and somatic cells thereof. Homozygote animals having the introduced DNA in both homogenous chromosomes are obtained, and these male and female animals can be crossbred to produce the offspring having said DNA and capable of breeding generation after generation.

The non-human mammals having the abnormal DNA of the present invention can be used as a morbid animal model since the abnormal DNA of the present invention is highly expressed in these animals to inhibit the functions of endogenous normal DNA, resulting in the onset of functional inactivated refractory conditions of the protein of the present invention. For example, the abnormal DNA-transferred animals of the present invention can be used to elucidate the mechanism of the morbid functional inactivated refractory conditions of the protein of the present invention or to examine the method of treating this disease.

For specific applicability, the abnormal DNA high-expression animals of the present invention serve as a model for elucidation of the functional inhibition (dominant negative action) of the normal protein by the abnormal protein of the present invention in the functional inactivated refractory conditions of the protein of the present invention. Further, the mammals having the extraneous abnormal DNA of the present invention transferred thereto can also be used in a test for screening a therapeutic agent against the functional inactivated refractory conditions of the protein of the present invention since these animals have the symptom of an increase in the protein of the present invention.

Other applicability of the two DNA-transferred animals of the present invention includes:

(1) Use thereof as a cellular source for tissue culture;

(2) Analysis of the relationship with proteins specifically expressed or activated by the protein of the present invention by direct analysis of mRNA in tissues in the DNA-transferred animals of the present invention or by analysis of tissues where the protein is expressed in the DNA-transferred animals;

(3) Screening of chemicals promoting or inhibiting the functions of cells by using the cells described according to item (1); and

(4) Isolation and purification of the variant protein of the present invention and preparation of an antibody against said protein.

Further, the DNA-transferred animals of the present invention can be used to examine the clinical symptoms of diseases correlated with the protein of the present invention, including the functional inactivated refractory conditions of the protein of the present invention, or to obtain further detailed pathological findings in each organ in a model with diseases correlated with the protein of the present invention, and to contribute to development of new therapeutic methods as well as to the study and treatment of secondary diseases resulting from said diseases.

Further, each organ is removed from the DNA-transferred animals of the present invention, cut into thin pieces, and digested with proteinase such as trypsin, whereby the free DNA-transferred cells can be obtained, and these cells can be cultured and these cultured cells can be established as a cell. line. By identifying those cells producing the protein of the present invention and by examining their relationship with differentiation or multiplication or the signal transfer mechanism therein, their abnormalities can be examined so that: these cells can be used as an useful material for study on the protein of the present invention and in elucidating their action.

The DNA-transferred animals of the present invention can also be used to provide an effective and rapid screening method for therapeutic agents for diseases correlated with the protein of the present invention by use of the examination method and quantification method described above, so that the therapeutic agents against these diseases including the functional inactive type refractory conditions of the protein of the present invention can be developed. Further, the DNA-transferred animals of the present invention or the extraneous DNA expression vector of the present invention can be used for examination and development of the gene therapeutic method to treat those diseases correlated with the protein of the present invention.

When bases or amino acids are expressed in abbreviations in the present specification and drawings, the following abbreviations in accordance with IUPAC-IUB Commission on Biochemical Nomenclature or based on customary abbreviations in this field are used. If amino acids can occur as optical isomers, L-isomers are referred to unless otherwise specified.

DNA: deoxyribonucleic acid
cDNA: complementary deoxyribonucleic acid
A: adenine
T: thymine
G: guanine
C: cytosine
RNA: ribonucleic acid
mRNA: messenger ribonucleic acid
dATP: deoxyadenosine triphosphate
dTTP: deoxythymidine triphosphate
dGTP: deoxyguanosine triphosphate
dCTP: deoxycytidine triphosphate
ATP: adenosine triphosphate
EDTA: ethylene diamine tetraacetate
SDS: sodium dodecyl sulfate
EIA: enzyme immunoassay
Gly: glycine
Ala: alanine
Val: valine
Leu: leucine
Ile: isoleucine
Ser: serine
Thr: threonine
Cys: cysteine
Met: methionine
Glu: glutamic acid
Asp: aspartic acid
Lys: lysine
Arg: arginine
His: histidine
Phe: phenyl alanine
Tyr: tyrosine
Trp: tryptophan
Pro: proline
Asn: asparagine
Gln: glutamine
pGl: pyroglutamic acid
Me: methyl group
Et: ethyl group
Bu: butyl group
Ph: phenyl group
TC: thiazolidine-4 (R)-carboxamide group

The substituent groups, protecting groups and reagents appearing frequently in the present specification are expressed in the following symbols.

Tos: p-toluene sulfonyl
CHO: formyl
Bzl: benzyl
$Cl_2$-Bzl: 2,6-dichlorobenzyl
Bom: benzyloxymethyl
Z: benzyloxycarbonyl

Cl-Z: 2-chlorobenzyloxycarbonyl
Br-Z: 2-bromobenzyloxycarbonyl
Boc: t-butoxycarbonyl
DNP: dinitrophenol
Trt: trityl
Bum: t-butoxymethyl
Fmoc: N-9-fluorenyl methoxycarbonyl
HOBt: 1-hydroxybenztriazole
HOOBt: 3,4-dihydro-3-hydroxy-4-oxo-1,2,3-benzotriazine
HONB: 1-hydroxy-5-norbornene-2,3-dicarbodiimide
DCC: N,N'-dicyclohexyl carbodiimide

The sequence numbers in the Sequence Listing in the present specification show the following sequences:

SEQ ID NO:1 shows the nucleotide sequence of a DNA fragment encoding an intracellular domain of activin IIA receptor protein as bait plasmid used in the two-hybrid method.
SEQ ID NO:2 shows the amino acid sequence of the protein of the present invention.
SEQ ID NO:3 shows the nucleotide sequence of a cDNA encoding the protein of the present invention having an amino acid sequence represented by SEQ ID NO:2.
SEQ ID NO:4 shows the nucleotide sequence of a DNA as a probe used in Northern hybridization.

The transformant Escherichia coli HB101/pACT2YA1 obtained in Example 1 below has been deposited under FERM BP-6927 from November 1, 1999 with the National Institute of Bioscience and Human-Technology (NIBH), the Agency of Industrial Science of Technology, the Ministry of International Trade and Industry, and under IFO 16325 with Institution Fermentation Organization (IFO) from October 14, 1999.
Hereinafter, the present invention is described in more detail by reference to the Examples, but is not limited thereto. The method of operating the two-hybrid method using a yeast was conducted according to instructions attached to the commercial kit (produced by Clontech), and the genetic manipulation using E. coli was conducted according to methods described in Molecular Cloning.

Example 1 Cloning of a cDNA encoding the protein of the present invention

(1) Screening of a protein interacting with activin IIA receptor protein by the two-hybrid method

For the following screening of a protein interacting with activin IIA receptor protein from a cDNA library, MATCHMAKER™ Two-Hybrid System 2 (Cat. No. K1604-1, Clontech) was used as a kit.
According to the method described above, an Eco RI site and Bam HI site in vector pAS2-1 for DNA ligation were cleaved with suitable restriction enzymes, treated with alkali phosphatase and then purified. Thereafter, a DNA fragment encoding an intracellular domain in the activin IIA receptor protein set forth in SEQ ID NO:1 was ligated thereto, to give a plasmid (pAS-IIA) expressing the desired GAL4 DNA-binding domain and activin IIA receptor intracellular domain as a fusion protein. As the GAL4 transcription-activating region-fused library plasmid, a commercial mouse brain MATCHMAKER cDNA library (Clontech) was used.
A single colony (diameter of 2 to 3 mm) of yeast strain Y190 was inoculated into 20 ml tryptophan-deficient SD medium and cultured under shaking for 18 hours at 30°C. 10 ml of this culture was inoculated into 300 ml YPD medium such that its $OD_{600}$ of 0.2 became 0.3, and the yeast was cultured under shaking at 30°C for 3 hours. The culture liquid was transferred to a sterilized centrifuge tube and centrifuged at 1000×g for 5 minutes at room temperature. The supernatant was discarded, and the cells were suspended in 25 ml sterilized water and centrifuged again at 1000×g for 5 minutes at room temperature. The supernatant was discarded, and the cells were suspended in a mixed solution of 1.5 ml TE buffer (0.01 M Tris-HCl, 1 mM EDTA, pH 7.5)/0.1 M lithium acetate (pH 7.5) and used for the subsequent transformation.
1 ml of the cell suspension prepared above was added to 10 μg of the previously prepared plasmid (pAS-IIA) and 2 mg herring spermary carrier DNA (Clontech), and well mixed. Further, 6 ml mixed solution of PEG (40% PEG 4000)/TE buffer/0.1 M lithium acetate was added thereto and mixed in a vortex mixer. After the mixture was shaken at 30°C at 200 rpm for 30 minutes, 700 μl DMSO (final concentration, 10%) was added thereto, and the mixture was stirred gently. Thereafter, the mixture was heated at 42°C for 15 minutes while being occasionally shaken, and after the container was cooled on ice, the mixture was centrifuged at 1000×g for 5 minutes. The supernatant was discarded, and the cells were suspended in 0.5 ml TE buffer. 100 μl of the resulting cell suspension was spread onto a tryptophan-deficient SD medium and cultured at 30°C for 4 days to give a strain maintaining said plasmid stably. Subsequently, the GAL4 transcription-activating region-fused library plasmid was also introduced in the same manner into said yeast strain. 0.2 ml of the resulting transformant was plated on a tryptophan/leucine/histidine-deficient SD medium and cultured at 30°C for 8 days, and $His^+$ colonies were streaked on a tryptophan/leucine/histidine-deficient agar plate.

5 ml mixture of buffer Z ($Na_2HPO_4 \cdot 7H_2O$, $NaH_2PO_4 \cdot H_2O$, KCl, $MgSO_4 \cdot 7H_2O$, pH 7)/X-gal solution (2% 5-bromo-4-chloro-3-indolyl-β-D-galactoside solution in DMF)/β-mercaptoethanol was placed in a Petri dish, to moisten a sterilized Whatman #5 filter therein. Another filter was placed on the agar plate containing said transformant colonies, and then this filter was removed and frozen in liquid nitrogen with the colonies facing upward. The filter was removed from liquid nitrogen and then thawed at room temperature, and with the colonies facing upward, the filter was placed on the previously moistened filter. A lid was placed on the Petri dish, and the colonies were incubated at 30°C for 1 hour, and about 100 blue positive colonies were separated therefrom. The resulting respective positive clones were inoculated into 3 ml leucine-deficient SD liquid medium and cultured for 2 days, and the culture liquid was diluted 10000-fold, plated on a leucine-deficient SD plate, and incubated at 30°C for 3 days. 20 to 30 colonies were picked up with a sterilized picker and replicated onto a tryptophan/leucine-deficient SD plate and a leucine-deficient SD plate. Then, those colonies requiring a tryptophan nutrient were selected from the previous positive colonies, and their β-galactosidase activity was assayed to select 2 colonies having no activity.

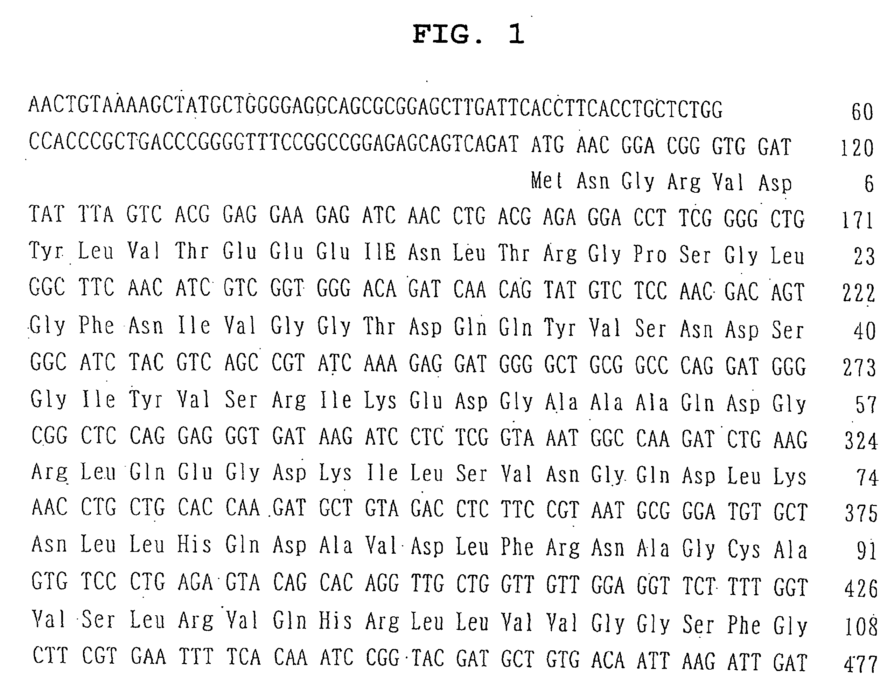

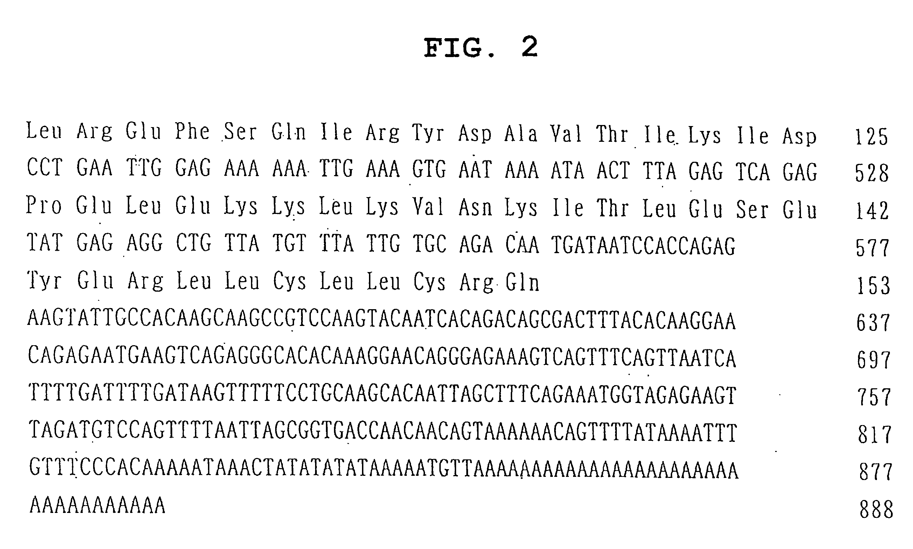

The resulting true positive colonies were inoculated into 2 ml YPD liquid medium and cultured at 30°C overnight. The culture liquid was centrifuged for 5 minutes at room temperature, and after the supernatant was discarded, 0.2 ml yeast-lyzing solution (2% Triton X-100, 1% SDS, 100 mM NaCl, 10 mM Tris-HCl (pH 8.0), 1 mM EDTA) was added to suspend the microorganisms. 0.2 ml phenol/chloroform/isoamyl alcohol (25 : 24 : 1) and glass beads washed with an acid were added thereto, and the mixture was stirred for 2 minutes with a vortex mixer. After centrifugation at 14000 rpm for 5 minutes at room temperature, the separated supernatant was mixed with a 1/10 volume of 3 M sodium acetate solution (pH 5.2) and a 2.5-fold excess volume of ethanol. The resulting precipitates were washed with 70% ethanol and dissolved in 20 μl sterilized water. 1 μl of this solution was introduced by electroporation into E. coli HB101, and the plasmid DNA was purified by the usual mini-prep method to give the desired cDNA (pACT2YA1) and determined the nucleotide sequence thereof. The full-length cDNA (pACT2YA1) for the protein of the present invention was 888 bp encoding a polypeptide [Figs. 1-2] consisting of 153 amino acids represented by SEQ ID NO:2.

Plasmid pACT2YA1 containing the full-length cDNA encoding a polypeptide consisting of amino acids represented by SEQ ID NO:2 was transformed into E. coli HB101, to give a transformant (E. coli HB101/pACT2YA1).

Example 2 Detection of expression by Northern hybridization using poly (A) $^+$RNA derived from a variety of mouse organs

The DNA fragments represented by SEQ ID NO:4 were labeled with digoxigenin by use of a DIG-PCR probe synthesis kit (Boehringer) and used as probes.

Further, the brain, liver, spleen, lung, kidney, hearts, testicles and skeletal muscles were excised from Balb/c mice, and total RNA was extracted therefrom with a TRIzol reagent (GIBCO BRL). Thereafter, poly (A) $^+$RNAs were purified by use of PolyAT tract mRNA Isolation System (Promega).

1 μg each of poly (A) $^+$RNAs was subjected to 1% agarose gel electrophoresis in a formalin-gel method and blotted onto Hybond N (Amersham-Pharmacia) by a vacuum blotting method using a blotting unit (Amersham-Pharmacia). The blotted sample was incubated at 65°C overnight in a hybridization buffer (5×SSC, 0.1% N-lauroylsarcosine, 0.02% SDS, 0.5% blocking reagent (Boehringer), 100 μg/ml salmon testicle DNA) containing 5 ng/ml of each of the previously prepared probe DNAs. Thereafter, the sample was washed 3 times with 0.1×SSC and 0.1% SDS at 65°C for 20 minutes. Further, the sample was maintained in a solution (0.1 M Tris-HCl pH 7.5, 0.15 M NaCl) containing 150 mU/ml alkali phosphatase-labeled anti-digoxigenin antibody (Boehringer), and then washed 3 times with 0.1 M Tris-HCl (pH 7.5), 0.15 M NaCl and 0.1% Tween 20 for 15 minutes. Finally, the sample was subjected to chemiluminescence using Lumi-Phos 530 (Wako Pure Chemical Industries, Ltd.) as the substrate and detected by exposure onto an X-ray film, and as a result, it was confirmed that a majority of the proteins of the present invention are highly expressed in the heart, the brain, the liver, the skeletal muscle, the kidney, the testis, etc.

## Industrial Applicability

The protein of the present invention, a partial peptide thereof or a salt thereof (also referred to hereinafter as the protein of the present invention), the DNA encoding the protein of the present invention or a partial peptide thereof (also referred to hereinafter as the DNA of the present invention), the antibody (also referred to hereinafter as the antibody of the present invention) against the protein of the present invention and the antisense DNA can be used as reagents in (1) determining a binding protein to the protein of the present invention, (2) obtaining an antigen and a serum, (3) construction of a system for expressing a recombinant protein, (4) development of a binding assay system using said expression system and an assay system using the two-hybrid method and screening of candidates for pharmaceutical compounds, (5) practice of a drug design based on comparison with structurally analogous ligand receptors and (6) formation of probes and PCR primers in gene diagnosis, etc., and further can be used as chemicals in (7) gene therapy, etc. In particular, elucidation of the structure and properties of the protein of the present invention leads to development of unique pharmaceutical preparations acting on these systems.

## Claims

1. A protein which has an amino acid sequence identical to or substantially identical to an amino acid sequence represented by SEQ ID NO:2, or a salt thereof.

2. A partial peptide of the protein according to claim 1, or a salt thereof.

3. A recombinant DNA which comprises a DNA encoding the protein according to claim 1.

4. The DNA according to claim 3, which has a nucleotide sequence represented by SEQ ID NO:3, or a nucleotide sequence hybridizing therewith under high stringent conditions.

5. A recombinant DNA which comprises a DNA encoding the partial peptide according to claim 2.

6. A recombinant vector comprising the DNA according to claim 3.

7. A transformant comprising the recombinant vector according to claim 6.

8. A method for producing the protein according to claim 1 or a salt thereof, which comprises culturing the transformant according to claim 7 to produce and accumulate the protein according to claim 1, and then recovering the product.

9. An antibody against the protein according to claim 1, the partial peptide according to claim 2 or a salt thereof.

10. A method for quantifying the protein according to claim 1, the partial peptide according to claim 2 or a salt thereof, which comprises allowing a test solution containing the protein according to claim 1, the partial peptide according to claim 2 or a salt thereof and the labeled protein according to claim 1, the labeled partial peptide according to claim 2 or a labeled salt thereof to react competitively with the antibody according to claim 9.

11. A method for determining a binding protein to the protein according to claim 1, the partial peptide according to claim 2 or a salt thereof, which comprises using the protein according to claim 1, the partial peptide according to claim 2 or a salt thereof.

12. A protein or a salt thereof which binds to the protein according to claim 1, the partial peptide according to claim 2 or a salt thereof, which is obtained by the method according to claim 11.

**13.** A method for screening a compound or a salt thereof inhibiting or promoting a binding of the protein according to claim 1, the partial peptide according to claim 2 or a salt thereof to the protein according to claim 12 or a salt thereof, or an activin receptor protein or a salt thereof, which comprises the protein according to claim 1, the partial peptide according to claim 2 or a salt thereof.

**14.** The method for determining a protein according to claim 11 or the screening method according to claim 13, which comprises using the two-hybrid method.

**15.** A kit for screening a compound or a salt thereof inhibiting or promoting a binding of the protein according to claim 1, the partial peptide according to claim 2 or a salt thereof to the protein according to claim 12 or a salt thereof, or an activin receptor protein or a salt thereof, which comprises the protein according to claim 1, the partial peptide according to claim 2 or a salt thereof.

**16.** A compound or a salt thereof which inhibits or promotes a binding of the protein according to claim 1, the partial peptide according to claim 2 or a salt thereof to the protein according to claim 12 or a salt thereof, or an activin receptor protein or a salt thereof, which is obtained by using the screening method according to claim 13 or by using the screening kit according to claim 15.

**17.** A medicament comprising the protein according to claim 12, the compound according to claim 16 or a salt thereof.

**18.** The medicament according to claim 17, which is an agent for preventing or treating an abnormality in a nerve cell or a cerebral disease correlated with the protein according to claim 12 or a salt thereof, an activin receptor or an activin intracellular information transmission molecule.

**19.** A method for preventing or treating an abnormality in a nerve cell or a cerebral disease correlated with the protein according to claim 12 or a salt thereof, an activin receptor or an activin intracellular information transmission molecule, which comprises administering an effective amount of the protein according to claim 12, the compound according to claim 16 or a salt thereof to a mammal.

**20.** Use of the protein according to claim 12, the compound according to claim 16 or a salt thereof for preparing an agent for preventing or treating an abnormality in a nerve cell or a cerebral disease correlated with the protein according to claim 12 or a salt thereof, an activin receptor or an activin intracellular information transmission molecule.

# FIG. 1

```
AACTGTAAAAGCTATGCTGGGGAGGCAGCGCGGAGCTTGATTCACCTTCACCTGCTCTGG        60
CCACCCGCTGACCCGGGGTTTCCGGCCGGAGAGCAGTCAGAT ATG AAC GGA CGG GTG GAT  120
                                           Met Asn Gly Arg Val Asp    6
TAT TTA GTC ACG GAG GAA GAG ATC AAC CTG ACG AGA GGA CCT TCG GGG CTG  171
Tyr Leu Val Thr Glu Glu Glu IlE Asn Leu Thr Arg Gly Pro Ser Gly Leu   23
GGC TTC AAC ATC GTC GGT GGG ACA GAT CAA CAG TAT GTC TCC AAC GAC AGT  222
Gly Phe Asn Ile Val Gly Gly Thr Asp Gln Gln Tyr Val Ser Asn Asp Ser   40
GGC ATC TAC GTC AGC CGT ATC AAA GAG GAT GGG GCT GCG GCC CAG GAT GGG  273
Gly Ile Tyr Val Ser Arg Ile Lys Glu Asp Gly Ala Ala Ala Gln Asp Gly   57
CGG CTC CAG GAG GGT GAT AAG ATC CTC TCG GTA AAT GGC CAA GAT CTG AAG  324
Arg Leu Gln Glu Gly Asp Lys Ile Leu Ser Val Asn Gly Gln Asp Leu Lys   74
AAC CTG CTG CAC CAA GAT GCT GTA GAC CTC TTC CGT AAT GCG GGA TGT GCT  375
Asn Leu Leu His Gln Asp Ala Val Asp Leu Phe Arg Asn Ala Gly Cys Ala   91
GTG TCC CTG AGA GTA CAG CAC AGG TTG CTG GTT GTT GGA GGT TCT TTT GGT  426
Val Ser Leu Arg Val Gln His Arg Leu Leu Val Val Gly Gly Ser Phe Gly  108
CTT CGT GAA TTT TCA CAA ATC CGG TAC GAT GCT GTG ACA ATT AAG ATT GAT  477
```

# FIG. 2

```
Leu Arg Glu Phe Ser Gln Ile Arg Tyr Asp Ala Val Thr Ile Lys Ile Asp    125
CCT GAA TTG GAG AAA AAA TTG AAA GTG AAT AAA ATA ACT TTA GAG TCA GAG    528
Pro Glu Leu Glu Lys Lys Leu Lys Val Asn Lys Ile Thr Leu Glu Ser Glu    142
TAT GAG AGG CTG TTA TGT TTA TTG TGC AGA CAA TGATAATCCACCAGAG           577
Tyr Glu Arg Leu Leu Cys Leu Leu Cys Arg Gln                            153
AAGTATTGCCACAAGCAAGCCGTCCAAGTACAATCACAGACAGCGACTTTACACAAGGAA           637
CAGAGAATGAAGTCAGAGGGCACACAAAGGAACAGGGAGAAAGTCAGTTTCAGTTAATCA           697
TTTTGATTTTGATAAGTTTTTCCTGCAAGCACAATTAGCTTTCAGAAATGGTAGAGAAGT           757
TAGATGTCCAGTTTTAATTAGCGGTGACCAACAACAGTAAAAAACAGTTTTATAAAATTT           817
GTTTCCCACAAAAATAAACTATATATATAAAAATGTTAAAAAAAAAAAAAAAAAAAAAAA           877
AAAAAAAAAAA                                                            888
```

## INTERNATIONAL SEARCH REPORT

International application No.
PCT/JP00/07480

A. CLASSIFICATION OF SUBJECT MATTER
Int.Cl[7] C12N15/12,C12N5/10,C12P21/02,C07K16/18,
C07K14/47,G01N33/50,G01N33/15,A61K38/17

B. FIELDS SEARCHED
Minimum documentation searched (classification system followed by classification symbols)
Int.Cl[7] C12N15/12,C12N5/10,C12P21/02,C07K16/18,
C07K14/47,G01N33/50,G01N33/15,A61K38/17

Documentation searched other than minimum documentation to the extent that such documents are included in the fields searched

Electronic data base consulted during the international search (name of data base and, where practicable, search terms used)
JICST FILE(JOIS),WPI(DIALOG),BIOSIS(DIALOG),
MEDLINE(STN),
EMBL/DDBJ/Genebank/PIR/Swissprot/Geneseq

C. DOCUMENTS CONSIDERED TO BE RELEVANT

| Category* | Citation of document, with indication, where appropriate, of the relevant passages | Relevant to claim No. |
|---|---|---|
| PX | EP, 1033401, A2 (Genset), 06 September, 2000 (06.09.00) Claims; (Sequence Nos. 3623, 7700) on the sequence list in the CD-ROM attached to the Gazette & CA, 2296792, A1 | 1-9 |
| X<br>Y | EMBO J., Vol.18 [11] (June 1999) Y. Nemoto et al. "Recruitment of an alternatively spliced form of synaptojanin 2 to mitochondria by the interaction with the PDZ domain of a mitochondrial outer membrane protein," pp.2991-3006, Figs. 1 A, 1D | 2,5,9,10<br>11-18,20 |
| X<br>Y | WO, 99/53051, A2 (Genset) 21 October, 1999 (21.10.99), Claims; sequence No. 769 & AU, 9930501, A | 2,5,9<br>10-18,20 |
| X<br>Y | WO, 98/40483, A2 (Human Genome Sci. Inc.) 17 September, 1998 (17.09.98), Claims; sequence Nos.20, 60 & AU, 9865521, A & EP, 973892, A1 | 2,5,9<br>10-18,20 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 November, 2000 (28.11.00) | 05 December, 2000 (05.12.00) |
| Name and mailing address of the ISA/<br>Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP00/07480

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | WO, 99/25825, A2 (Genset),<br>27 May, 1999 (27.05.99)<br>Claims; sequence Nos. 48,140,142<br>& AU, 9910491, A & EP, 1029045, A1 | 2,5,9<br>10-18,20 |
| X<br>Y | WO, 99/31326, A2 (Genset),<br>24 July, 1999 (24.07.99)<br>Claims; sequence No. 135<br>& AU, 9915030, A & EP, 1037977, A1 | 2,5,9<br>10-18,20 |
| Y | EP, 943684, A2 (Smithkline Beecham P.L.C.),<br>22 September, 1999 (22.09.99)<br>& JP, 11-253183, A & CA, 2249661, A1 | 10-18,20 |
| Y | EP, 937777, A2 (Smithkline Beecham P.L.C.),<br>25 August, 1999 (25.08.99),<br>& JP, 11-253182, A & CA, 2249653, A1<br>& US, 6043054, A | 10-18,20 |
| Y | EP, 933425, A2 (Smithkline Beecham P.L.C.),<br>04 August, 1999 (04.08.99)<br>& JP, 11-225778, A & CA, 2246001, A1 | 10-18,20 |

Form PCT/ISA/210 (continuation of second sheet) (July 1992)

**INTERNATIONAL SEARCH REPORT**

International application No.
PCT/JP00/07480

**Box I Observations where certain claims were found unsearchable (Continuation of item 1 of first sheet)**

This international search report has not been established in respect of certain claims under Article 17(2)(a) for the following reasons:

1. ☒ Claims Nos.: 19
because they relate to subject matter not required to be searched by this Authority, namely:

The invention as set forth in claim 19 pertains to methods for prevention or treatment of diseases and thus relates to a subject matter which this International Searching Authority is not required, under the provisions of Article 17(2)(a)(i) of the PCT and Rule 39.1(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)

SEQUENCE LISTING

<110>Takeda Chemical Industries, Ltd.

<120>Novel Protein and Its Use

<130>2657WOOP

<150>JP11-305187

<151>1999-10-27

<150>JP11-313453

<151>1999-11-04

<160>4

<210>1

<211>1442

<212>DNA

<213>Mouse

<400>1

AGACATCACA AGATGGCCTA CCCTCCTGTA CTTGTTCCTA CTCAAGACCC AGGACCACCC 60
CCACCTTCCC CATTACTAGG GTTGAAGCCA TTGCAGCTGT TAGAAGTGAA AGCAAGGGGA 120
AGATTTGGTT GTGTCTGGAA AGCCCAGTTG CTCAATGAAT ATGTGGCTGT CAAAATATTT 180
CCAATACAGG ACAAACAGTC CTGGCAGAAT GAATATGAAG TCTATAGTCT ACCTGGAATG 240
AAGCATGAGA ACATACTACA GTTCATTGGT GCAGAGAAAA GAGGCACCAG TGTGGATGTG 300
GACCTGTGGC TAATCACAGC ATTTCATGAA AAGGGCTCAC TGTCAGACTT TCTTAAGGCT 360
AATGTGGTCT CTTGGAATGA ACTTTGTCAT ATTGCAGAAA CCATGGCTAG AGGATTGGCA 420
TATTTACATG AGGATATACC TGGCTTAAAA GATGGCCACA AGCCTGCAAT CTCTCACAGG 480
GACATCAAAA GTAAAAATGT GCTGTTGAAA AACAATCTGA CAGCTTGCAT TGCTGACTTT 540
GGGTTGGCCT TAAAGTTCGA GGCTGGCAAG TCTGCAGGTG ACACCCATGG GCAGGTTGGT 600
ACCCGGAGGT ATATGGCTCC AGAGGTGTTG GAGGGTGCTA TAAACTTCCA AAGGGACGCA 660
TTTCTGAGGA TAGATATGTA CGCCATGGGA TTAGTCCTAT GGGAATTGGC TTCTCGTTGC 720
ACTGCTGCAG ATGGACCCGT AGATGAGTAC ATGTTACCAT TTGAGGAAGA AATTGGCCAG 780
CATCCATCTC TTGAAGATAT GCAGGAAGTT GTTGTGCATA AAAAAAAGAG GCCTGTTTTA 840
AGAGATTATT GGCAGAAACA TGCAGGAATG GCAATGCTCT GTGAAACGAT AGAAGAATGT 900

TGGGATCATG ATGCAGAAGC CAGGTTATCA GCTGGATGTG TAGGTGAAAG AATTACTCAG 960
ATGCAAAGAC TAACAAATAT CATTACTACA GAGGACATTG TAACAGTGGT CACAATGGTG 1020
ACAAATGTTG ACTTTCCTCC CAAAGAATCT AGTCTATGAT GGTGGCACCG TCTGTACACA 1080
CTGAGGACTG GGACTCTGAA CTGGAGCTGC TAAGCTAAGG AAAGTGCTTA GTTGATTTTC 1140
TGTGTGAAAT GAGTAGGATG CCTCCAGGAC ATGTACGCAA GCAGCCCCTT GTGGAAAGCA 1200
TGGATCTGGG AGATGGATCT GGGAAACTTA CTGCATCGTC TGCAGCACAG ATATGAAGAG 1260
GAGTCTAAGG GAAAAGCTGC AAACTGTAAA GAACTTCTGA AAATGTACTC GAAGAATGTG 1320
GCCCTCTCCA AATCAAGGAT CTTTTGGACC TGGCTAATCA AGTATTTGCA AAACTGACAT 1380
CAGATTTCTT AATGTCTGTC AGAAGACACT AATTCCTTAA ATGAACTACT GCTATTTTTT 1440
TT 1442

<210>2
<211>153
<212>PRT
<213>Mouse
<400>2

Met Asn Gly Arg Val Asp Tyr Leu Val Thr Glu Glu Glu IlE Asn Leu
1 5 10 15
Thr Arg Gly Pro Ser Gly Leu Gly Phe Asn Ile Val Gly Gly Thr Asp
20 25 30
Gln Gln Tyr Val Ser Asn Asp Ser Gly Ile Tyr Val Ser Arg Ile Lys
35 40 45
Glu Asp Gly Ala Ala Ala Gln Asp Gly Arg Leu Gln Glu Gly Asp Lys
50 55 60
Ile Leu Ser Val Asn Gly Gln Asp Leu Lys Asn Leu Leu His Gln Asp
65 70 75 80
Ala Val Asp Leu Phe Arg Asn Ala Gly Cys Ala Val Ser Leu Arg Val
85 90 95
Gln His Arg Leu Leu Val Val Gly Gly Ser Phe Gly Leu Arg Glu Phe
100 105 110

Ser Gln Ile Arg Tyr Asp Ala Val Thr Ile Lys Ile Asp Pro Glu Leu
115 120 125
Glu Lys Lys Leu Lys Val Asn Lys Ile Thr Leu Glu Ser Glu Tyr Glu
130 135 140
Arg Leu Leu Cys Leu Leu Cys Arg Gln
145 150 153

<210>3
<211>459
<212>DNA
<213>Mouse
<400>3

```
ATGAACGGAC GGGTGGATTA TTTAGTCACG GAGGAAGAGA TCAACCTGAC GAGAGGACCT   60
TCGGGGCTGG GCTTCAACAT CGTCGGTGGG ACAGATCAAC AGTATGTCTC CAACGACAGT  120
GGCATCTACG TCAGCCGTAT CAAAGAGGAT GGGGCTGCGG CCCAGGATGG GCGGCTCCAG  180
GAGGGTGATA AGATCCTCTC GGTAAATGGC CAAGATCTGA AGAACCTGCT GCACCAAGAT  240
GCTGTAGACC TCTTCCGTAA TGCGGGATGT GCTGTGTCCC TGAGAGTACA GCACAGGTTG  300
CTGGTTGTTG GAGGTTCTTT TGGTCTTCGT GAATTTTCAC AAATCCGGTA CGATGCTGTG  360
ACAATTAAGA TTGATCCTGA ATTGGAGAAA AAATTGAAAG TGAATAAAAT AACTTTAGAG  420
TCAGAGTATG AGAGGCTGTT ATGTTTATTG TGCAGACAA                         459
```

<210>4
<211>888
<212>DNA
<213>Mouse
<400>4

```
AACTGTAAAA GCTATGCTGG GGAGGCAGCG CGGAGCTTGA TTCACCTTCA CCTGCTCTGG   60
CCACCCGCTG ACCCGGGGTT TCCGGCCGGA GAGCAGTCAG ATATGAACGG ACGGGTGGAT  120
TATTTAGTCA CGGAGGAAGA GATCAACCTG ACGAGAGGAC CTTCGGGGCT GGGCTTCAAC  180
ATCGTCGGTG GGACAGATCA ACAGTATGTC TCCAACGACA GTGGCATCTA CGTCAGCCGT  240
ATCAAAGAGG ATGGGGCTGC GGCCCAGGAT GGGCGGCTCC AGGAGGGTGA TAAGATCCTC  300
```

```
TCGGTAAATG GCCAAGATCT GAAGAACCTG CTGCACCAAG ATGCTGTAGA CCTCTTCCGT  360
AATGCGGGAT GTGCTGTGTC CCTGAGAGTA CAGCACAGGT TGCTGGTTGT TGGAGGTTCT  420
TTTGGTCTTC GTGAATTTTC ACAAATCCGG TACGATGCTG TGACAATTAA GATTGATCCT  480
GAATTGGAGA AAAAATTGAA AGTGAATAAA ATAACTTTAG AGTCAGAGTA TGAGAGGCTG  540
TTATGTTTAT TGTGCAGACA ATGATAATCC ACCAGAGAAG TATTGCCACA AGCAAGCCGT  600
CCAAGTACAA TCACAGACAG CGACTTTACA CAAGGAACAG AGAATGAAGT CAGAGGGCAC  660
ACAAAGGAAC AGGGAGAAAG TCAGTTTCAG TTAATCATTT TGATTTTGAT AAGTTTTTCC  720
TGCAAGCACA ATTAGCTTTC AGAAATGGTA GAGAAGTTAG ATGTCCAGTT TTAATTAGCG  780
GTGACCAACA ACAGTAAAAA ACAGTTTTAT AAAATTTGTT TCCCACAAAA ATAAACTATA  840
TATATAAAAA TGTTAAAAAA AAAAAAAAAA AAAAAAAAAA AAAAAAAA              888
```